# EUROPEAN PATENT SPECIFICATION

(11) **EP 3 505 218 B1**
(45) Date of publication and mention of the grant of the patent: **25.01.2023**
(21) Application number: 18211568.3
(22) Date of filing: 11.12.2018
(51) Int. Cl.: A61P 17/10, A61Q 19/00, A61Q 19/10, A61K 8/44, A61K 8/39, A61K 8/42

(54) **A GENTLE DETERGENT COMPOSITION FOR GREASY AND ACNEIC SKINS**
SANFTE REINIGUNGSZUSAMMENSETZUNG FÜR FETTIGE UND AKNEISCHE HAUT
COMPOSITION DOUCE DETERGENTE POUR PEAUX GRASSES ET ACNEIQUES

(30) Priority: 13.12.2017 IT 201700143871
(43) Date of publication of application: 03.07.2019
(73) Proprietor: Unifarco S.p.A., 32035 Santa Giustina (BL) (IT)
(72) Inventor: Baratto, Giovanni, 32035 Santa Giustina BL (IT); Semenzato, Alessandra, 32035 Santa Giustina BL (IT); Costantini, Alessia, 32035 Santa Giustina BL (IT)
(74) Representative: Perani & Partners S.p.A.

(56) References cited:
- EP-A2- 2 682 161
- US-A1- 2008 070 986
- US-A1- 2016 324 743
- Anonymous: "CAE", , 1 February 2016 (2016-02-01), XP055484014, Retrieved from the Internet: URL:http://www.aminoscience.com.br/cosmeti cos/tensoativos/CAE-E-160201_nomark.pdf [retrieved on 2018-06-13]
- DATABASE GNPD [Online] MINTEL; 29 July 2016 (2016-07-29), anonymous: "Acne Foaming Cleanser", XP55591421, retrieved from www.gnpd.com Database accession no. 4172863

## Description

### FIELD OF THE INVENTION

The present invention relates to a gentle detergent composition for the treatment of greasy skin and of acneic skin.

### STATE OF THE ART

The skin, especially on the face, is one of the most sensitive parts of the human body, the most exposed and the one that reacts more to external stimuli, to emotions and to the reactions that they arouse. Therefore, the importance of keeping it intact, elastic and hydrated must not be underestimated.

Sebaceous secretion is involved in the maintenance of these properties. It contributes to the formation of the surface hydrolipid film that protects the skin from chemical and bacterial aggressions and is an important factor of thermoregulation and hydration, since it controls the epidermis loss of water.

There are different types of skin, differing from each other based on the balance among the elements that compose them, namely water, proteins and fats, as well as to external and genetic conditions. The different types of skin are generally classified based on these parameters as normal, dry, greasy, mixed and sensitive. These types of skin can in turn have variants.

Normal skin is a widely used term referring to well-balanced skin, scientifically called eudermic. It appears luminous, evidently tonic and elastic, smooth to the touch with a clear and rosy complexion and therefore well vascularized. It is the one that shows a harmonious relationship among its structural components.

Dry skin, on the other hand, is defined as the one in which the percentage of aqueous content is reduced compared to normal parameters and its characteristic appearance is conditioned by the reduced sebaceous component of the hydrolipid mantle due to an insufficient lipid secretion. To the touch it is rough, cracked, not very elastic and irregular, with scales. This injury occurs as a result of several factors: an increase in the epidermal cell turnover perhaps due to a subclinical inflammatory state, a loss of water from the stratum corneum or a damage to the barrier with an alteration of keratinocytes and a decrease in the ability to retain water. Dryness can also be acquired or resulting from exposure to UV rays or to aggressive chemicals. This type of skin therefore requires intense hydration with the reintegration of lipids compatible with those of the stratum corneum, such as cholesterol. The dehydrated skin favours the penetration of external material, with possible onset of inflammatory forms of irritative or allergic contact.

Greasy skin, on the other hand, appears shiny and greasy to the touch due to an excessive sebum production, often has enlarged pores and tends to reddening. The production of sebum can be normalized by using products containing matting, adsorbing and antibacterial agents. It is also important to pay a particular attention to the use of not too aggressive detergents, since aggressive detergents could cause the so-called "paradoxical effect" whereby the sebaceous glands respond to the insult suffered by the skin with an increase in sebum production. The evolution of sebum overproduction can result in a recurrent multifactorial dermatosis, better known as acne. Acne Vulgaris is a multifaceted skin disorder attributed to various pathogenic factors that cause the formation of acneic lesions.

For a long time a primary responsibility has been attributed to sebum overproduction (mediated by androgens), considering inflammation as the final event in its pathogenesis (Tanghetti A., The Role of Inflammation in the Pathology of Acne, The Journal of Clinical and Aesthetic Dermatology (2013), volume 6, 9: 27-35). It has been shown instead how the inflammatory phenomena are present in the perifollicular site already from the first stages of development of acneic lesions, to the point that today inflammation can be considered as a possible primary event of the pathogenetic acneic cascade (Taylor M., Gonzalez M., Porter R., Pathways to inflammation: acne pathophysiology, European Journal of Dermatology (2011), 21 (3): 323-33.).

Thanks to the increased sebum secretion, the acneic patient should not apparently suffer from evident signs of skin dehydration. However, some data suggest that the increased sebum secretion can alter the balance of the barrier function of the skin, and therefore the balance of skin hydration (Yamamoto A., Takenouchi K., Ito M., Impaired water barrier function in acne vulgaris, Archives of Dermatological Research (1995), 287 (2): 214-218). The dilution performed by the sebaceous lipids against the epidermal ones contributes to the alterations of keratinization at the infrainfundibole level of the sebaceous follicle, leading to a swelling of the follicular wall with the formation of open or closed comedones and therefore with the obstruction of the pilosebaceous outlet on the skin surface. Among lipids of epidermal origin, the lack of ceramides and sphingolipids in particular is considered of primary importance in the pathogenesis of this disorder and in the alteration of the skin barrier function.

Moreover, a gram-positive bacterium known as *Proprionobacterium Acnes* proliferates within the ducts of the pilosebaceous follicles. It acts in anaerobiosis by means of a lipase that hydrolyses the triglycerides in the sebum into free fatty acids, which are extremely irritating to the follicles, thus initiating an intense flogogenic activity supported by bacterial enzymes and inflammatory cytokines. As a result, the skin becomes inflamed and reddened.

*Propionobacterium Acnes* appears to be a member of the human microbiome even in those who do not develop an acneic pathology. In fact, the colonization of the bacterium on the skin is a necessary but not sufficient condition for the establishment of this dermatological disorder (Webster, 2001). There are various predisposing or aggravating causal factors for acne, namely genetic predisposition, immuno/inflammatory and hormonal factors (Chen WC., Zouboulis CC., Hormones and the pilosebaceous unit, Dermato- Endocrinology (2009), 1 (2): 81- 6.).

Acne treatment is largely symptomatic, namely aiming to mitigate the symptoms, since the triggering factors are multiple and not yet fully known.

Cleansing is considered an essential adjuvant process in this disorder. It aims at rebalancing the activity of the sebaceous glands but also at hydrating the superficial layers, fighting bacterial proliferation and preparing the skin for the absorption of any active ingredients by removing the surface cells (Subramanyan K., Dermatologic Therapy (2004), 17 (1): 26-34.).

A common tendency is cleansing the skin with products made of aggressive surfactants that can cause a rebound effect, thus favouring the production of sebum and increasing skin irritation. Aggressive surfactants can cause damage to the skin barrier. In fact, they increase the trans-epidermal water loss factor (TEWL), the bacterial colonization, the pH alteration and the formation of comedones, thus leaving a skin that is irritated and therefore more predisposed to this pathology (Levin J., The Relationship of Proper Skin Cleansing to Pathophysiology, Clinical Benefit, and the Concomitant Use of Prescription Topical Therapies in Patients with Acne Vulgaris, Dermatologic Clinics (2016), 34 (2): 133-45).

Therefore, taking care of your personal hygiene is without a doubt a good habit, but if excessive or practiced by using aggressive products, it can become a practice harmful to patients with problems of greasy, acne-prone skin. To avoid this paradox, cleansers for acneic skin should not contain any aggressive surfactants that give a basic pH to the skin, thus keeping the human microbiome intact.

The mixed skin is the one that groups, in the same integument, non-contiguous areas of normal skin and greasy skin.

The term sensitive skin, on the other hand, means a set of objective and subjective symptoms that are not yet fully standardized and identified. It shows a condition of susceptibility, reactivity and intolerability to external agents.

The term "cleansing" refers to that act of cosmetic hygiene aimed at removing from the surfaces of our body (skin, mucous membranes and hair) the so-called exogenous dirt, deriving from environmental and/or endogenous contamination characterized by sebaceous secretions. Since the products intended for this purpose are cosmetics, they fall within the legislative definition (Law Reg. 1223/2009) of substances and preparations, other than medicines, intended to be applied on the external surfaces of the human body or on the teeth and on mucous membranes of the mouth for the exclusive or prevailing purpose of cleaning them, perfuming them, changing their appearance, correcting their odour, protecting them or keeping them in good condition.

The ideal product for skin hygiene should remove dirt in a gentle way thanks to the presence of surfactants (primary functionality), respecting the integrity of the skin through the restoration of the lipid barrier (secondary function) thanks to lipids, vegetable proteins or quaternary derivatives such as conditioning agents.

Cleansing can take place through two mechanisms of action: by contrast or by affinity.

When cleansing by affinity, the substance responsible for the detergent action is lipophilic, like an oil or lipid with the addition of emulsifiers, which incorporate by affinity into the matrix the dirt that is mechanically removed, thus acting gently on the skin. This type of cleansing is definitely the most suitable way to respect the normal structure and physiology of the skin, because the main components mainly play an emollient and film-forming function, well indicated for dry skin. The cleansing products acting by affinity, however, do not have foaming properties. In order to have this latter property, foaming detergent emulsions consisting of a lipid substance combined with a gentle surfactant that may require rinsing or not are available on the market.

Contrast cleansing, on the other hand, requires only the use of surfactants, which together with the water eliminate the dirt by means of a rinsing action. It is preferable for cleansing greasy and acneic skin, even if it is more irritating to the skin if compared to affinity cleansing. The surfactants, acting on the water surface tension (force present at the contact between water and air) and on the interfacial tension (force present at the contact between two liquids) facilitate the phenomena of wettability, emulsification and removal of fat substances, which normally do not disperse in water.

Cleansing is the most common phenomenon through which this process of removal and dissolution of the dirt that accumulates during the day takes place.

The parameter that expresses the washing capacity of a surfactant, and therefore also its potential aggressiveness, is indicated with the name of active washing substance (SAL), that is the percentage of pure surfactant present in the formulation.

The washing capacity of a product therefore depends on the chemical structure of the used surfactants, on their mixing ratio and on the concentration of the active washing substance of the single products.

The calculation of SAL is the first approach for the formulation of a detergent product and must take into account its functional characteristics (type of skin/hair to be washed) and the area of use on which the product is to be applied.

If a detergent is intended for use on the mucous membranes, such as detergents for intimate hygiene, the SAL of the product must be around 10%, shampoo and face cleansers usually have a SAL between 10-20%, whereas bubble baths, which are used at high concentrations and on the whole body, can reach an active washing substance of 30-35%. Emollient and fatting agents are often inserted in the formulations of products to be applied on the whole body containing high concentrations of surfactants, these agents being able to restore the barrier lipids depleted by washing.

Generally, the detergent formulation technology has been characterized by the creation of systems that better respect the skin barrier and currently provides three strategic approaches:
- reducing the damage caused by an excessive detergency by using **surfactants with gentle intrinsic characteristics**, e.g. having an amino acid base or consisting of a polar head large enough to not allow the surfactant to penetrate the lipid double layer;
- compensating the damage caused by surfactants by introducing **moisturizing factors** such as glycerine or lactates known for their ability to retain water in the skin barrier. The barrier lipids could also be restored with fatty acids and sterols, but these latter can be easily removed after the product has been rinsed (Yang L., Vincent C., Ananthapadmanabhan KP, Lips A., Enhancing mildness of Syndet Cleansing Bars, Journal of the American Academy of Dermatology (2005), 52 (3): 37). Cationic polymers can be used to increase their deposition on the skin. However, this strategy does not prevent any barrier damage caused during cleaning but simply masks the resulting injuries;
- **providing positive benefits** through the use of barrier repairers, nutrients and conditioners able to restore the normal function of the skin barrier.

Surfactants are the main components of detergents. They are amphipathic substances consisting of a hydrophobic portion connected to a polar hydrophilic portion, which can be ionic, non-ionic or amphoteric.

Because of this double component, these molecules show a strong tendency to migrate towards the interface between two phases: they orient the polar head group in a water phase and the apolar one in a hydrophobic phase (dirt), thus decreasing the surface tension of a liquid and increasing the wetting power of the surfaces they come in contact with.

Based on their chemical structure, they therefore perform different functions:
➢ Solubilizers: ability to disperse an insoluble liquid in another liquid though maintaining its transparency.
➢ Foaming agents: ability to produce foam. The foam is due to the surfactant agent present in the product, which forms an emulsion of air in water, and is therefore a dispersed system consisting of gas bubbles in a liquid. The foam helps to disperse the particles of dirt, facilitates their removal and avoids their new precipitation on the washed surface. The foaming power depends on the nature of the surfactant.
➢ Emulsifiers: ability thereof to be placed at the interface between two immiscible liquids, forming adsorbed layers that prevent coalescence.
➢ Bactericides: antimicrobial property, mainly of cationic surfactants.
➢ Detergents: ability to remove insoluble substances through the use of lipophilic chains. The surfactant micelles subsequently incorporate this insoluble particle and expose the hydrophilic part to the water, which then allows the removal of the dirt from the skin surface.
➢ Conditioners: adsorption of a cationic molecule on a negatively charged surface (often become such after cleansing). In the case of conditioning agents without charge, the conditioning action is instead given by the establishment of hydrophobic bonds between them and the proteins present on the skin and/or the hair.

The surface activity and the various applications of surfactants are determined by the balance between the hydrophilic and lipophilic portion of the molecule, called HLB value, which is based on their molecular weights.

Since the ratio between the molecular weights of the hydrophilic portion and the whole molecule varies between 0 (prevailing hydrophobic content) and 1 (only hydrophilic part), it follows that the HLB ranges between 0 and 20. Therefore molecules with HLB > 10 are predominantly hydrophilic, whereas those with HLB < 10 are lipophilic. In particular, those with an HLB between 0 and 3 work as antifoaming agents, between 4 and 6 as W/O emulsifiers, between 7 and 9 as wetting agents, between 8 and 16 as O/W emulsifiers, between 13 and 15 as detergents, between 10 and 18 as solubilizers as reported in the following Table 1.

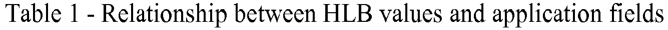

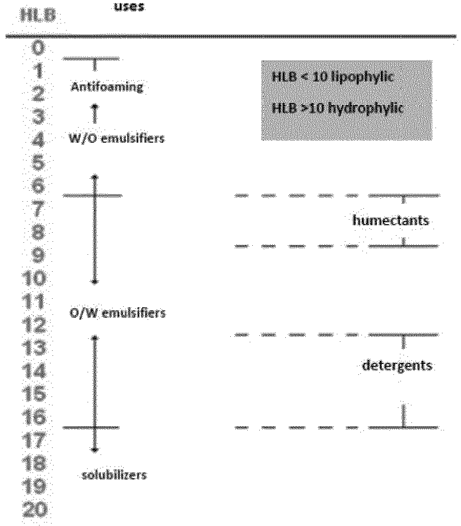

When introduced into water, surfactants are oriented with respect to the surface so as to remove the hydrophobic groups from the aqueous phase and reach a minimum energy state. The attractive forces between these groups and the water molecules are smaller than those existing between the water molecules: the ability of the surface to be contracted is therefore reduced, and consequently the surface tension is lowered too. Once the saturation of the liquid surface has been reached, the surfactant molecules decrease the surface tension and dissociate into micellar formations in which they expose to the solvent their related fraction and subtract to the solvent their non-related fraction. They have a tendency to break the hydrogen bonds between the existing water molecules by placing themselves around the non-polar entity of the molecule, thus restoring the bonds.

The surfactant concentration at which such micelles begin to form is referred to as critical micellar concentration (CMC) and is typical of any surfactant once exceeded the Krafft temperature (critical micellar temperature).

In particular, the critical micellar concentration linearly decreases with the increase of the hydrophobicity of the surfactant system, whereas surfactants with prevailing polar groups have a higher CMC. This is due to the repulsion between adjacent surfactant heads and to the increase in surfactant solubility in water. Electrolytes also reduce the CMC of cleaning solutions, since the repulsion between similar charges is reduced and they structure the system by exposing the hydrophobic groups. The ramifications and unsaturations of double bonds between carbon atoms cause an increase in the CMC compared to the linear chain compound. With the same hydrocarbon chain the ionic surfactants, and therefore those having a charge, have higher CMC values comprised between 10⁻³ and 10⁻² M, whereas non-ionic surfactants have a critical micellar concentration at room temperature comprised between around 10⁻⁴ and 10⁻⁵ M.

Mixtures of different surfactants chemically originate micelles at a lower concentration than that the one needed for the individual surfactant. For this reason, detergents are never formulated with a single surfactant but with a mixture of them, since the association reduces the single CMC, the micelles are formed at a lower concentration, and proportionally, the irritating action is reduced too.

The micellization process is spontaneous and upon reaching the critical micellar concentration, the addition of a further surfactant does not influence the surface tension. The shape of the micelles can be spherical, cylindrical, lamellar or vesicular, and the prevalence of one of them depends on temperature, concentration, nature of the surfactant and electrolytes present in the system.

Another parameter that characterizes surfactants is the aggregation number, namely the number of amphiphilic molecules forming the micelle and useful to describe the micelle size. In particular, in an aqueous solution, the greater the hydrophobic chain of a surfactant, the higher the aggregation number, or it may increase as the hydrophilicity of the polar head decreases. Moreover, a reduction in hydrophilicity as a high concentration of electrolytes causes an increase in the aggregation number, whereas high temperatures reduce it.

The surfactants can be classified as ionic and non-ionic according to their chemical dissociation in water. Cationic, anionic and zwitterionic surfactants belong to the first class, whereas the neutral ones belong to the second class.

The most commonly used surfactants and their characteristics are reported in the following table 2.

**Table 2 of the most used commercial surfactants and their characteristics**

| Categories | Classes | Properties |
|---|---|---|
| STRONG ANIONIC surfactants | Sulfuric esters: alkyl sulphates | Excellent washing and foaming agents, inexpensive but aggressive. Ethoxylation reduces aggression and increases solubility in water |
| WEAK ANIONIC surfactants | Alkyl ethers sulphates | Ethoxylation reduces aggression and increases water solubility |
| | Sulphonates and sulphosuccinates | Well tolerated but with limited foaming power |
| | Alkyl heterocarboxylates | Excellent dermatological properties, poor foaming properties. |
| | Protein condensates with fatty acids or amino acid derivatives: acyl sarcosinates, acyl glutamates, acyl methyl taurates, acyl peptides, acyl isethionates | They reduce the irritative potential of other surfactants. Well tolerated. |
| CATIONIC surfactants | Quaternary ammonium salts | Non-detergent but substantiating action on negatively charged substrates. Used as bactericides and conditioners |
| AMPHOTERIC surfactants | Alkyl betaines and alkyl sulfobetaines | Dermocompatible and reducing the irritative potential of anionics. Viscosizing agents, excellent foaming power |
| NON IONIC surfactants | Esters of polyalcohols and glycerol Esters of sorbitol ethoxylates Alkylpolyglucosides Esters of sucrose with fatty acids Alkanolamides | Good washing agents, scarcely foaming Insensitive to pH changes Good viscosizing and dispersing agents |
| | | |
| | | |

A fraction of the dermatological problems related to skin irritation may be related to the skin high exposure to surfactant solutions.

The ability of surfactants to absorb at the interface can in fact produce desirable or undesirable effects on the skin depending on the concentration of the surfactant, the type of exposure, the contact duration and the individual response.

To formulate a detergent system that respects the skin barrier it is therefore necessary to understand how surfactants alter the lipid bilayer.

The stratum corneum consists of corneocytes inserted into an intercellular lipid matrix composed of ceramides, long chain fatty acids, cholesterol and cholesterol esters. This particular physical conformation of the lipids allows creating a semipermeable barrier to the passage of water through the tissue (Rawlings AV, Scott IR, Harding CR, Bowser PA, Stratum corneum moisturization at the molecular level, Journal of Investigative Dermatology (1994), 103 (5): 731-741.).

Only recently, toxicological researches and dermatological controls have initiated a critical re-examination of the prolonged cutaneous use of detergents containing surfactants, highlighting their physiological limits.

In fact, surfactants exert their damaging action on the skin in various ways.
➢ They dissolve the hydrolipid film by solubilizing the lipids in their micelles, removing them physically or increasing their fluidity. In particular, they alter the lipid composition of the skin barrier (Imokawa G., Akasaki S., Minematsu Y., Kawai M., Importance of intercellular lipids in the water-retention properties of the stratum corneum: Introduction and recovery study of surfactant dry skin, Archives of Dermatological Research (1989), 281 (1): 45-51).
➢ Thus, they increase the permeability of the barrier and destabilize it by inserting themselves between the intercomeocytic lipids. It has been recently demonstrated that the penetration capacity of a surfactant such as sodium dodecyl sulphate in the epidermis is related to its total concentration, given by the presence of both monomers and micelles in solution. In particular, the addition of the polyethylene oxide (PEO) polymer to the detergent system containing sodium dodecyl sulphate increases the micelle radius from 2 nm to 2.5 nm thanks to its steric hindrance (Moore PN, Puwada S, Blankschtein D., Challenging the surfactant monomer skin penetration model: penetration of sodium dodecyl sulphate micelles into the epidermis, Journal of Cosmetic Science (2003), 54 (1): 29-46; Moore, PN, Shiloach, A., Puwada, S., Blankschtein, D ., Penetration of mixed micelles into the epidermis: Effect of mixing sodium dodecyl sulphate with dodecyl hexa (ethylene oxide), Journal of Cosmetic Science (2003), 54 (2): 143-159). In fact, thanks to their amphiphilic nature, the micelles can penetrate into the stratum corneum through the aqueous pores arranged between the intercellular lipids whose radius has a size between 1 and 2.8 nm (Peck KD, Hsu J., Li Sk, Ghanem AH., Higuchi WI, Flux enhancement effect of ionic surfactant upon passive and electroosmotic transdermal transport, Journal of Pharmaceutical Science (1998), 87: 1161-1169.). It is therefore easy to understand how micelles with a larger diameter can hardly interfere in the stratum corneum: increasing the diameter of the micelles could turn out to be a possible strategy to decrease the irritating potential of surfactants.
➢ Anionic surfactants, in particular the sulforic ones, promote the dissolution of proteins (e.g. keratin) in the stratum corneum, thus causing the release of sulfhydryl groups that react with various enzymes present on the skin: they are in fact considered as major skin irritants if compared to others with the same detergent power. Non-ionic surfactants, on the other hand, bind to proteins through a weak hydrophobic interaction and are therefore more skin-compatible. They are in fact often used in combination with the anionic ones to minimize damage (Ananthapadmanabhan KP., Subramanyan K., Rattiger GB., Moisturizing Cleaners. Leyden JJ., Rawlings AV., Eds., Skin Moisturization, Cosmetic Science and Technology Series (2002), 25: 406). The surfactants then cause a transient swelling phenomenon by binding to the proteins: the damages produced in the conformation of the keratins are such as to determine first phenomena of hyperhydration and swelling of the corneocyte, and then a deep dehydration. Many studies have shown that surfactants with a larger polar head size have a lower tendency to cause denaturing of proteins and therefore create less barrier damage (Pierard GE., Goffin V., Pierard-Franchimont C., Corneosulfametry: a predictive assessment of the personal care cleansing product with human stratum corneum, Dermatology (1994), 189 (1): 152-6et al., 1994).
➢ Surfactants remove low molecular weight hydrophilic factors such as the natural hydration factor (NMF), minimizing the ability to retain water from the skin and causing changes in skin elasticity (Prottey C., Ferguson T., Factors which determine the skin irritation potential of soap and detergents, Journal of the Society of Cosmetic Chemists (1975), 26 (1): 29-46.).
➢ Surfactants degrade the intercomeocytic enzymatic systems essential for the proper organization of lamellar lipids (A-SMase, beta-glucocerebrosidase) that undergo irreversible alterations. This action is due to the direct protein denaturation capacity expressed by the surfactant and by an excess of endogenous protease activity.
➢ The surfactants damage the same vital cells of the granulose layer in which the detergents affect the membranes and compromise the lipid synthesis.
➢ Deteriorating the barrier, the surfactants compromise its ability to inhibit the penetration of exogenous substances, thus laying the basis for the development of a hyperactivity of sensitive skin or of a true contact dermatitis (Terranova F., physiopathology of skin hydration, New Techniques, Milan (2006).
➢ Surfactants can also induce the release of inflammatory cytokines, in particular interleukin 1-α, after their interaction with the stratum corneum: they are however released in conditions of dysfunction of the skin barrier to stimulate its repair (Gibbs S., Vietsch H., Meier U., Ponec M., Effect of skin barrier competence on SLS and water-induced IL-I alpha expression, Experimental Dermatology (2002), 11 (3): 217-23). In a skin that has already been irritated by surfactants, however, this inflammatory process causes an overstimulation and an increase in the corneification rate that does not allow a complete reform of the stratum corneum in a rapid time. The cells therefore remain fragile and lose their barrier properties and the natural factor of hydration thus remaining even more susceptible to the aggressive action of surfactants (Wickett RR, Visscher MO, Structure and function of epidermal barrier, American Journal of Infection Control (2006), 34 (10): 98-110).

The challenge launched by the formulator is breaking this vicious circle in some way and restoring the barrier lipids through the insertion of emollients.

This last approach, however, has some limitations when taking into consideration the treatment of a greasy and acneic skin. In fact, since there is already a sebum overproduction, it is not indicated to insert occlusive lipophilic agents that would further worsen the disorder.

In fact, seborrheic skins, often subjected to over-cleansing, are more affected by the aggression of surfactants and may suffer more due to the loss of lipid and water-soluble factors that cannot be immediately restored. This creates a vicious circle of damage to the skin.

The need to overcome this drawback is therefore felt, for example, by selecting mixtures of intrinsically gentle surfactants that minimize the "upstream" damage and by inserting conditioning agents and/or rheological modifiers capable of restoring the skin optimal charge conditions.

The conditioning activity of a detergent product is due to the presence of compounds with a positive charge and without charge. In order to exercise its function, the conditioning agent must be adsorbed on the skin surface. This property, defined as substantivity, depends on intrinsic factors of the molecule, such as electrical charge, ratio between polar and apolar groups, molecular weight and skin surface.

The adsorption of the cationic molecule on keratin, a protein present on the skin surface, neutralizes its negative charges. This property is very sought after in the formulations of detergents for greasy skin as it allows bringing the skin back to optimal charge conditions, thus improving the after-feel of the wash. In the case of cleansing-intended formulations, it is important to consider the charge density of the substance, since the compatibility with the anionic surfactants depends on it. For example, *Cetrimonium chloride* cannot be used in a detergent based on *Sodium Laureth Sulfate.* In contrast, *Polyquaternium-7* is the most common conditioner used in shampoos, thanks to its lower charge density and complete compatibility with anionic surfactants. Cationic conditioners can be classified into non-polymeric and polymeric molecules (D'Agostinis G., Mignini E., Manuale del cosmetologo: Ricerca applicata, progettazione, engineering, produzione, marketing, packaging, discipline collegate, Tecniche nuove, Milan (2006)).

Non-polymeric cationic conditioning agents are molecules with surfactant/emulsifying activity. Their charge density, and therefore their ability to bind skin keratins, are higher in the presence of short alkyl chains. Many conditioners belonging to this category have the common name of *Quaternium* followed by a number: the higher the number, the more recent is the introduction of the molecule on the market (e.g. *Quaternium-12*).

Polymeric cationic conditioning agents are obtained by quaternization of natural polymers (polysaccharides and proteins) or by synthesis. They are very versatile from the point of view of the application, even if they have a lower conditioning power if compared to non-polymeric quaternary conditioner. They have a longer prolonged film-forming effect over time, are incompatible with anionic surfactants and can sometimes be over-applied on the skin (e.g. Polyquaternium-10). In recent years, a group of modified natural substances have been developed, produced by condensation between a quaternary ammonium group and protein derivatives. They are generally water-soluble, compatible with anionics and provided with a high skin tolerability (e.g. *Hydroxypropyltrimonium Hydrolysed Protein*).

In the case of conditioners with no charge, however, the substantivity on the skin is due to the establishment of hydrophobic bonds between them and keratin. The non-cationic conditioners provide in their formulation the use of fatty alcohols (cetyl alcohol and stearyl alcohol), whose function is not only to provide consistency to the formulation, but also to condition the skin. Among them, there are also several amino acid derivatives, such as those used in this work (e.g. *Lauroyl Arginine*). Rheological modifiers in detergents not only contribute to the viscosity of the product, but are essential for improving the physical-chemical stability among the components of a surfactant mixture. Viscosizing agents further allow the direct application of the product on the skin. In fact, if the detergent is too fluid, it could be difficult to spread and could therefore not be appreciated by the consumer or could possibly be overdosed. The main categories of rheological modifiers on the cosmetic market are electrolytes, alkanolamides and polyethylene glycol derivatives.

The most used are electrolytes, in other words inorganic salts such as sodium chloride or organic sodium lactate, since they are the most comfortable systems, have a high yield and are less expensive. Electrolytes create interactions with the surfactant system due to the chemical characteristics of the electrolyte. They favour the structuring of surfactant aggregates and lead to a structural change of the micelles that take a spherical shape. However, they are free to move and thus create a low-viscosity system. In fact, the ions surrounded by solvation water are outside the surfactant micelles, thus increasing their size and making the system more compact. In fact, they reduce the repulsion between the charges and the effective area of the polar head. However, there is a limitation in the concentration of electrolyte TO BE added to the detergent system above which there is a new viscosity decrease: the excessive concentration of salt, in fact, causes a disorganization of the micellar structure.

The addition of this substance can sometimes, in some surfactant systems, raise the *cloud point* of the mixture, thereby reducing its transparency.

The second category of viscosizing agents used in the cosmetic field are alkanolamides: they are acylation products of various alkanolamines, i.e. fatty acids. Their viscosizing action requires the presence of electrolytes and these derivatives are also used as foam supporters. Among the most common ones there are *Cocamide DEA* (diethanolamide) and *Cocamide MEA* (monoethanolamide): they can give rise to the formation of nitrosamines in detergent products that cause skin irritations (Turkoglu M., Pekmezci E., Sakr A., Evaluation of irritation potential of surfactant mixtures, International Journal of Cosmetic Science (1999), 21 (6): 371-82.). The alkanolamides, when mixed with primary surfactants, globally reduce the polar group area forming typical rod-shaped micelles suitable for increasing the viscosity of the detergent.

If systems containing surfactants such as sulphates and betaine are easily viscosized, this is not the case when the product is formulated with high doses of gentle surfactants. In this context, salts and alkanolamides are often ineffective and are substituted by molecules with a high molecular weight, generally obtained by esterification or etherification of polyethylene glycol chains (D'Agostinis and Mignini, cited above). In spite of a very high efficacy, the use of excessive amounts of these derivatives can cause a feeling of stickiness after the application of the product to the skin. The polyethylene glycol derivatives consist of a small polar head and of long lipophilic tails that are positioned within the micellar structure forming a *network* thanks to intermolecular associations, thus increasing the micelle stability and size. The hydrophobic segments are able to adsorb on the surface of the particles dispersed in the system, hence the name of "associative" viscosizing agents (Braun D.B., Rosen M.R., Rheology Modifiers Handbook, practical use and application, New York, (2000)). In particular, the number of non-ionic hydrophobic tails is directly related to the efficiency by which they create viscosity in formulations with gentle surfactants.

For example, *PEG-150 distearate* has two hydrophobic groups and can bind few surfactant micelles, thus only slightly increasing the viscosity of the system. At high doses, it tends to break down the foam of the detergent system. Subsequently viscosizing agents with three or four hydrophobic arms consisting of oleic and stearic acid derivatives have been developed. Among these there are *PEG-200 hydrogenated glyceryl palmate* with three hydrophobic arms and *PEG-120 methyl glucose trioleate*, which has four arms as shown in the following formula (I)

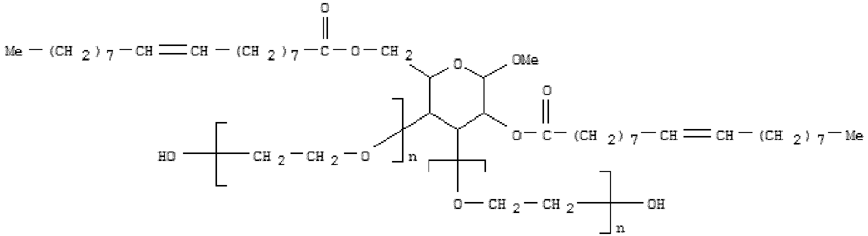

Chemically speaking, *PEG-120 methyl glucose trioleate* is a methyl glucose ethoxylate ether esterified with oleic acid.

Another viscosizing agent used in this study is *PEG-55 propylene glycol oleate*, which has the chemical structure shown in the following formula (II). It is a polyethylene glycol ether of propylene glycol derived from oleic acid.

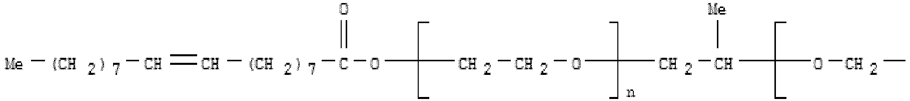

The high length of these molecules, favouring the cross-linking of the system, causes a dose-dependent viscosity increase. These derivatives should not be used in solutions with too an acidic or alkaline pHs, as a hydrolysis of the ester group could occur, resulting in a loss of viscosity. The molecules of this group are used at low concentration percentages (maximum 3 to 4%) and can also act as fatting agents, thus reducing the aggressiveness of any lauryl ether sulphate present. The fatting effect, however, restricts the field of use of these derivatives: in the formulation of detergents for greasy skin, in fact, this property can lead to a worsening of the disorder.

Further simpler yet equally effective molecules are available on the market, such as *Laureth-2* and *Laureth-3*, which are an alternative to alkanolamides. Polymers, on the other hand, are used when traditional viscosizing agents are ineffective and when a system capable of suspending particles is required. The polymers do not determine an increase in micelles, but they structure the water: the presence of lipophilic portions ensures an interaction with the micelles. In the cosmetic field, there are few effective polymers, since the high ionic strength of the medium disturbs the formation of the polymeric network. Among them are *Xanthan Gum*, acrylic latexes and cellulose.

In this perspective, the Applicant conducted a research to identify a detergent composition with an activity that can be advantageously used for cleansing greasy skin and in particular acneic skin. A study was carried out on mixtures of intrinsically gentle surfactants with a conditioning and/or a rheological action.

The study was carried out in successive steps.
- In the first step, a mixture of gentle surfactants was selected and characterized by dynamic light scattering analysis and viscosimetric analysis in order to determine the optimal ratio between surfactants maintaining a 15% active washing substance (SAL).
- The contribution of two conditioning agents, *Lauroyl Arginine* and *PCA ethyl cocoyl arginate* was then evaluated in terms of viscosity and stability with the mixture components with 15% of SAL
- Then, the active washing substance (SAL) was reduced up to 12.5% and 10%.
- In the last step, viscosizing agents of different chemical nature were added to make the detergent system more stable and able to guarantee its applicability on the skin. The behaviour of non-ionic electrolytes and thickeners in surfactant mixtures with different SALs in terms of viscosity and micelle size was monitored through dynamic light scattering analysis.

The following documents represent related prior art documents.

DATABASE GNPD (online) MINTEL;29 July 2016(2015-07-29) Anonymous "Acne foaming cleanser" retrieved from www.gnpd.com Database accession no.4172863, discloses a cleansing composition of acneic skin wherein sodium cocoyl alaninate, is one of the several substances present in said composition This compound is not considered as essential, since the efficacy of this composition for the specific use in cleansing acneic skin is the presence of: hyaluronic acid, vitamin B5, witch hazel, aloe and isopropylmethylphenol.

Anonymous CAE 1 February 2016 (2016-02-1) Retrieved from the internet URL:http//www.aminoscience.com.br/cosmeticos/tensioativos/CAE-e-160201_nomark.pdf describes the ability of component of PCA ethyl cocoyl arginate as: an antibacterial agent against Proppioni bacterium acnes, as a biodegradable component in increasing hair resiliency.

EP2682161 discloses a liquid cleansing composition containing a long listo of aminoacidic surfactants, and among them also sodium cocoylalaninate is encompassed.

US20160324743 discloses a composition for coloring and shampooing hair, , substantially free from anionic surfactants comprising at least one cationic dye and an N-methyl-alkyl-glucamide.

US2008/0070986 discloses that Laureth 7-citrate is 2 magnitude orders less effective as antibacterial against Propionibacterium acnes, if compared with other surfactants (see table 1 page 1 right column). In order to increase this antibacterial property it suggests to add considerable amounts of the very aggressive sodium laureth sulfate.

### SUMMARY OF THE INVENTION

The Applicant has surprisingly found that of all the detergent compositions, only the following composition has the desired requisites and therefore constitutes the object of the present invention. It comprises:
a) sodium cocoyl alaninate;
b) cocoyl methyl glucamide;
c) laureth-7 citrate, and
d) PCA ethyl cocoyl arginate;
in which:
i) a, b, c arc the cleaning substances and the amount of a + b + c is between 8 and 18% by weight on the total weight of said composition;
ii) the ratio of (a/b/c)/c) is between 0.8 and 1.4;
iii) d is present in an amount ranging from 0.1 to 0.6% by weight on the total weight of said composition.

The detergent compositions may contain in addition to the aforesaid active ingredients, excipients and/or diluents, such as e.g. pH regulators, aqueous solvents, flavouring substances and/or perfumes.

This composition is particularly suitable for cleansing greasy and acneic skin.

### DESCRIPTION OF THE FIGURES

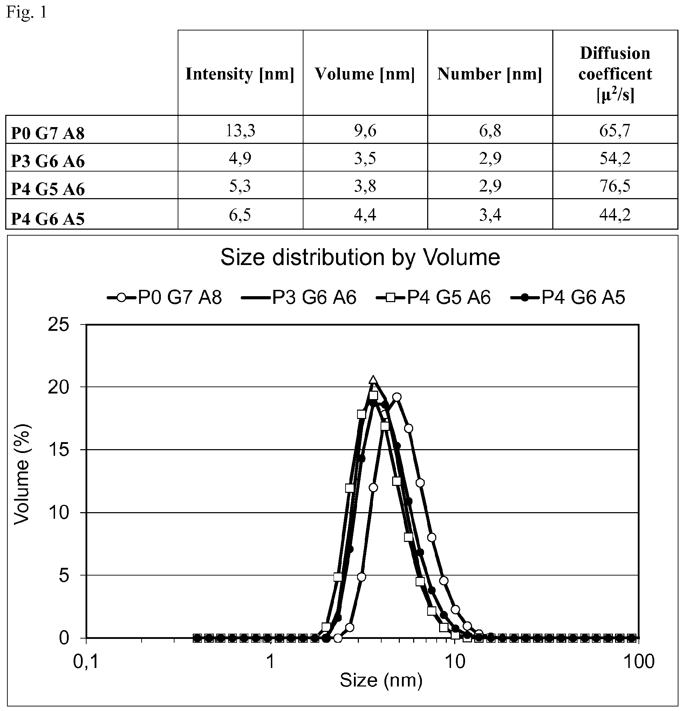

Figure 1: the first figure is the table showing the mean micellar diameter values obtained by dynamic light scattering analysis expressed as intensity, volume and number of samples in different ratios with 15% SAL. The second figure is a graph of the distribution of the micellar volumetric diameter of samples in the same ratios of the first figure with 15% SAL.

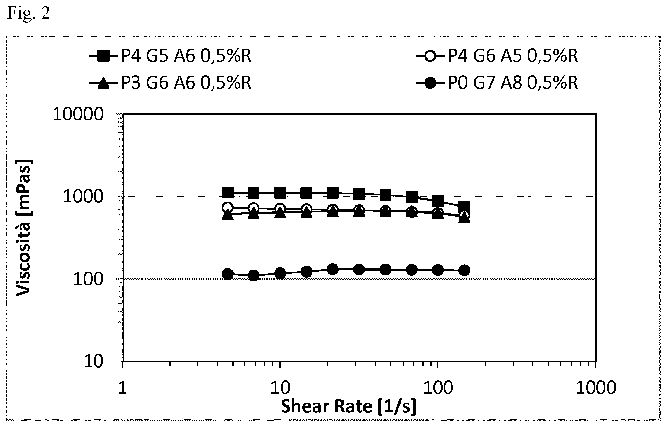

Figure 2: shows the graph of the viscosity trend as a function of the sample shear rate in the same ratios of Figure 1 with 15% SAL containing 0.5% w/w sodium chloride (R).

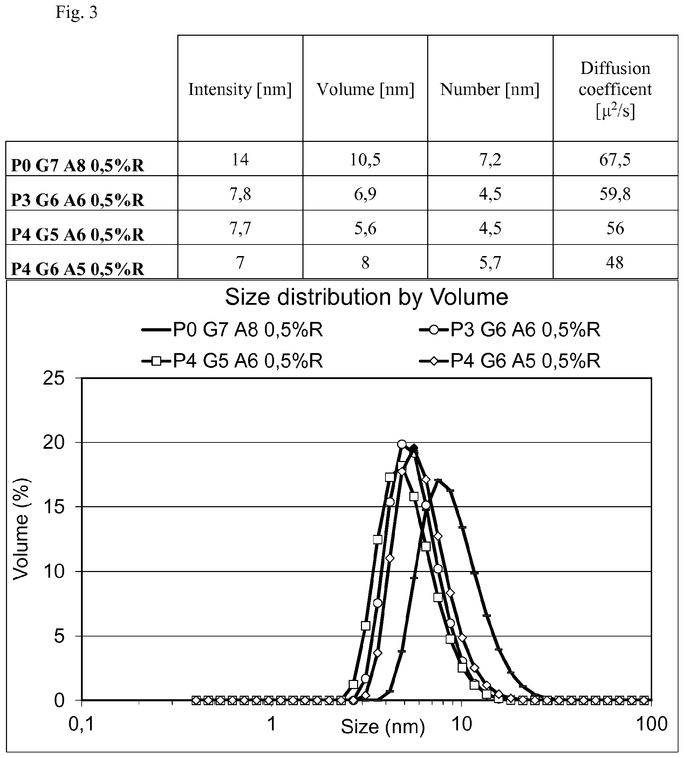

Figure 3: the first figure is the table showing the mean micellar diameter values obtained by dynamic light scattering analysis expressed in the form of intensity, volume and number of samples in different ratios with 15% SAL and 0.5% w/w sodium chloride (R). The second figure is a graph of the distribution of the micellar volumetric diameter of samples in the same ratios with 15% SAL and 0.5% w/w sodium chloride.

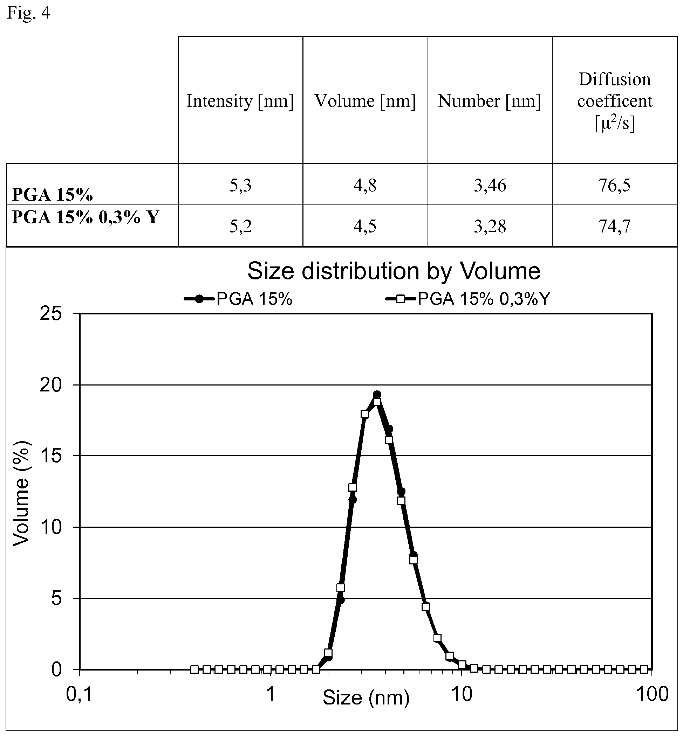

Figure 4: the first figure is the table showing the values of the mean micellar diameter obtained by dynamic light scattering analysis expressed in the form of intensity, volume and number of 15% PGA and 15% PGA, 0.3% w/w lauroyl arginine (Y). The second figure shows the volumetric distribution curves of the same formulations.

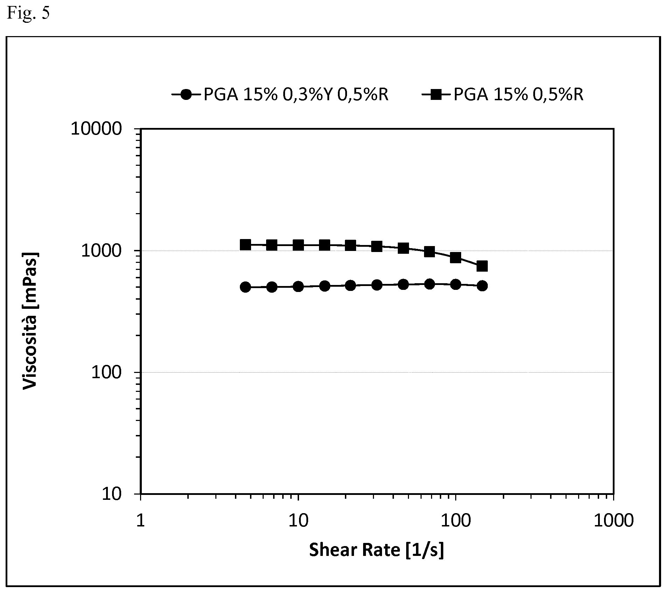

Figure 5: shows the graph of the viscosity trend as a function of the shear rate of 15% PGA, 0.3% w/w lauroyl arginine (Y), 0.5% w/w sodium chloride (R), 15% PGA and 0.5% sodium chloride.

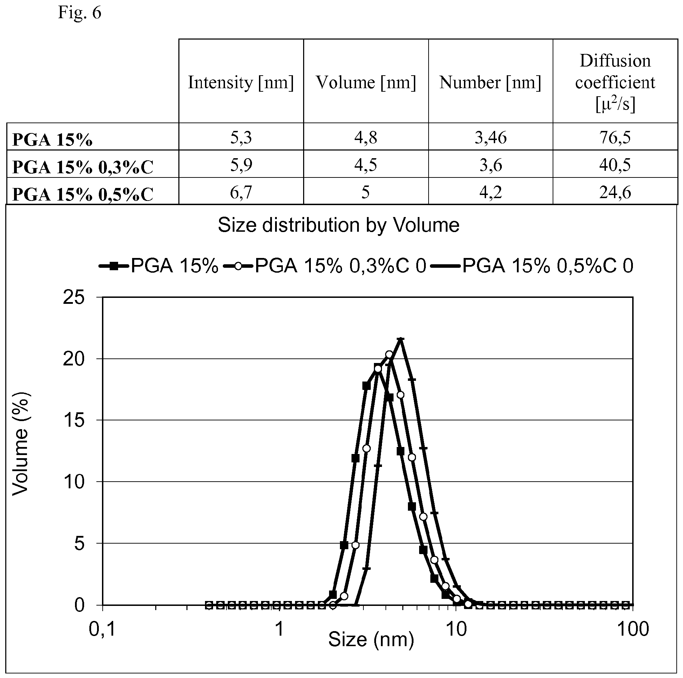

Figure 6: the first figure is the table showing the values of the mean micellar diameter obtained by dynamic light scattering analysis expressed in the form of intensity, volume and number of the PGA sample (15%) with increasing concentrations of PCA ethyl cocoyl arginate (C). The second figure shows a graph of the volumetric distribution of the same formulations.

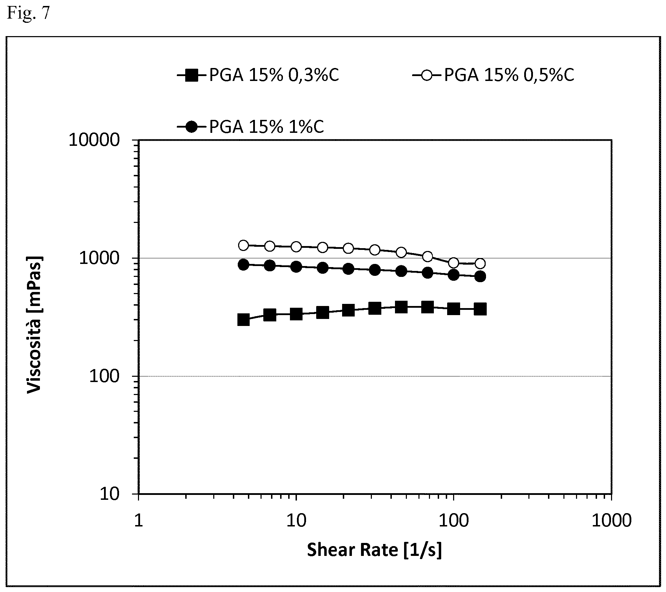

Figure 7: shows the graph of the viscosity trend as a function of the shear rate of 15% PGA with increasing concentrations of (C).

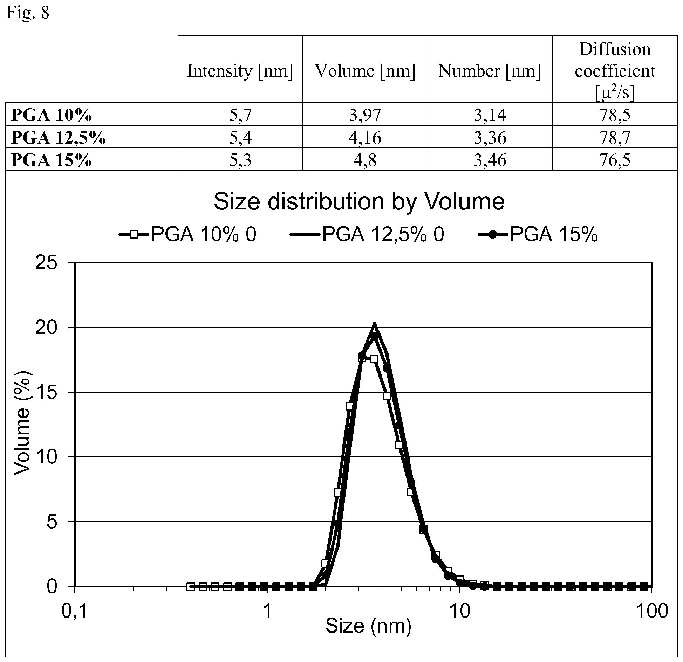

Figure 8: the first figure is the table of mean micellar diameter values obtained by dynamic light scattering analysis expressed in the form of intensity, volume and number of samples with active washing substance (SAL) of 10% (10% PGA), 12.5% (12.5% PGA) and 15% (15% PGA). The second figure shows in graph the volumetric distribution of the same formulations.

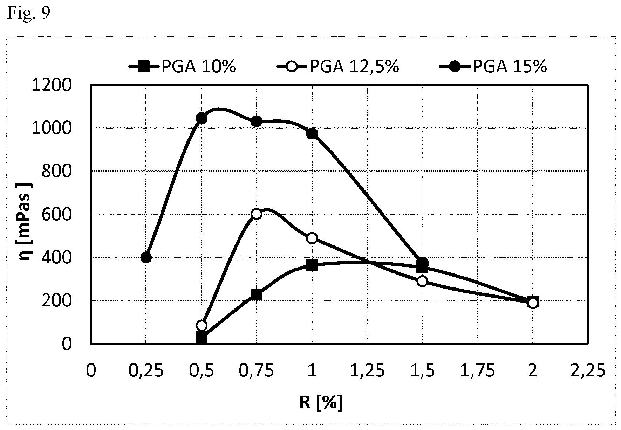

Figure 9: shows the graph of the viscosity trend at a shear rate of 46.4 s⁻¹ of 10% PGA, 12.5% PGA and 15% PGA and increasing sodium chloride (R) concentrations.

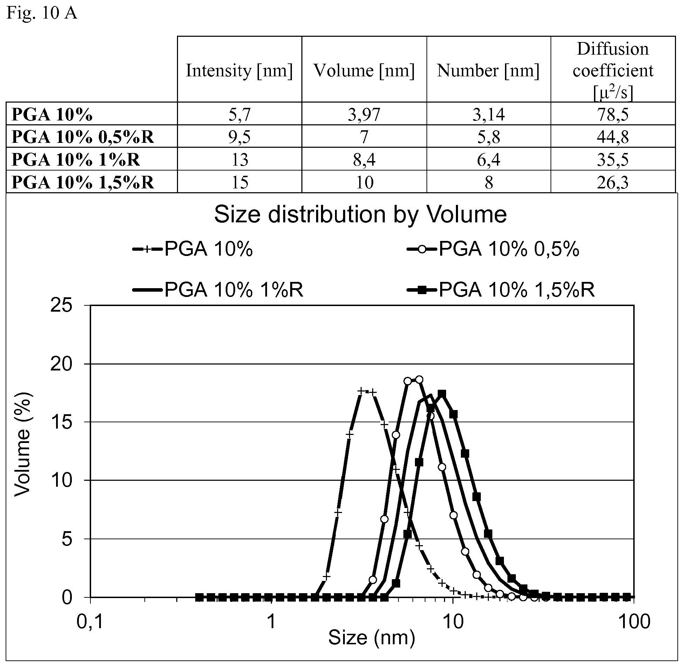

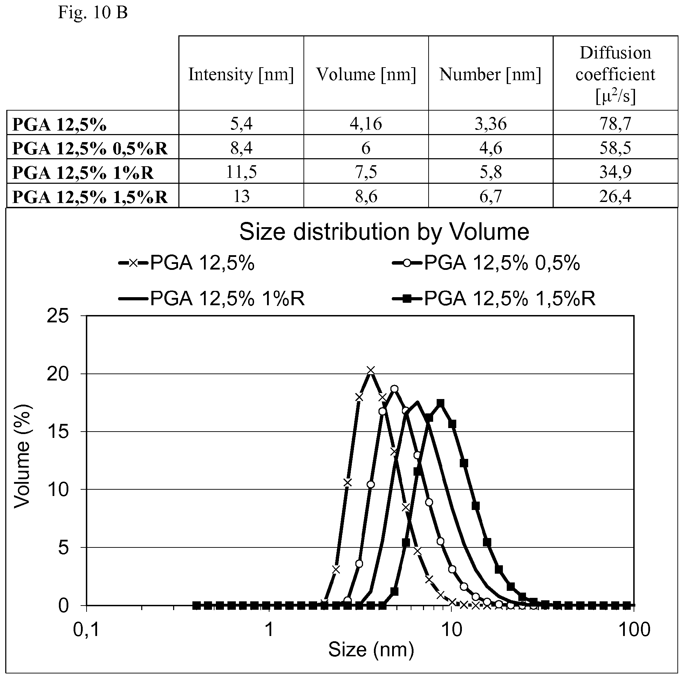

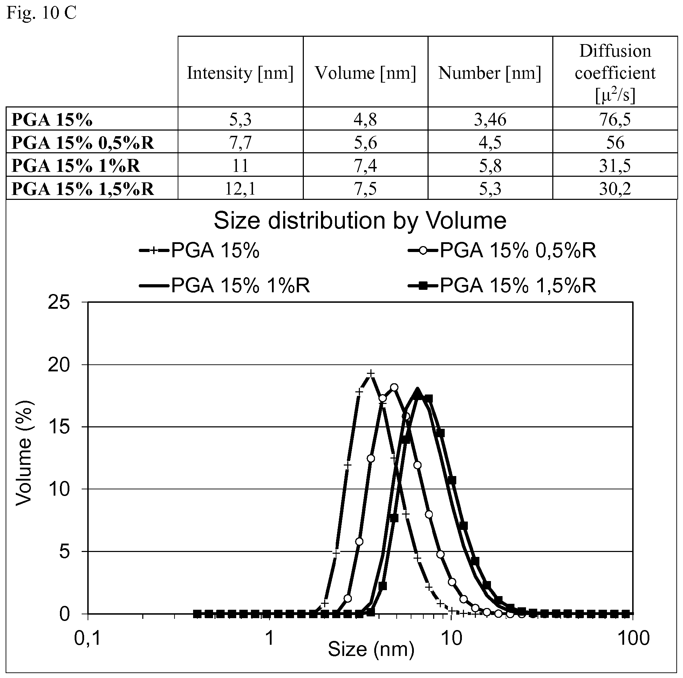

Figures 10A-C: the first figures show the mean micellar diameter values obtained by dynamic light scattering analysis expressed in the form of intensity, volume and number of samples respectively of 10% PGA, 12.5% PGA and 15% PGA with increasing concentrations of sodium chloride (R). The second figures show the volumetric distribution curves of the same formulations.

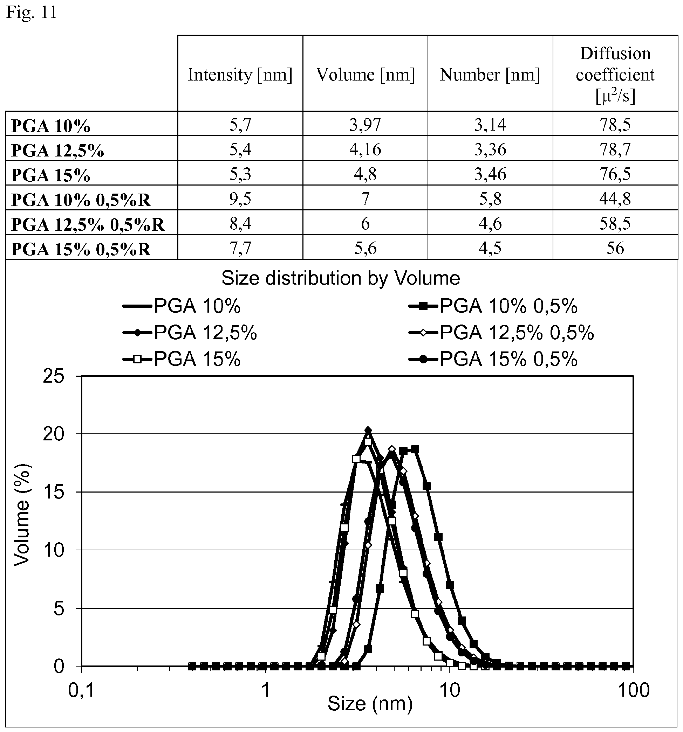

Figure 11: the first figure shows the mean micellar diameter values obtained by dynamic light scattering analysis expressed in intensity, volume and number of samples of 10% PGA, 12.5% PGA and 15% PGA with 0.5% w/w sodium chloride (R). The second figure shows in graph the volumetric distribution of the same formulations.

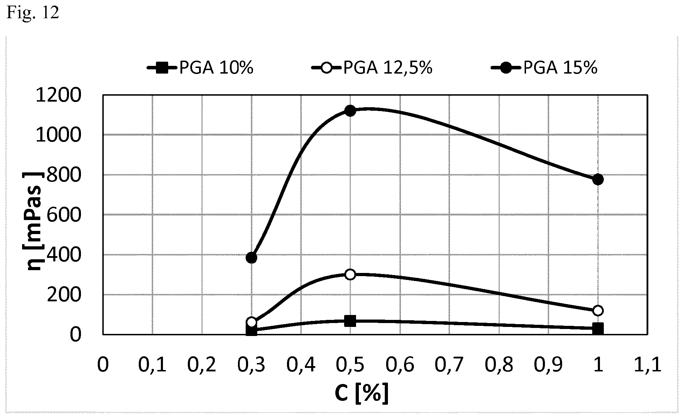

Figure 12: shows the graph of the viscosity trend at a shear rate of 46.4 s⁻¹ of 10% PGA, 12.5% PGA and 15% PGA and with increasing concentrations of PCA ethyl cocoyl arginate.

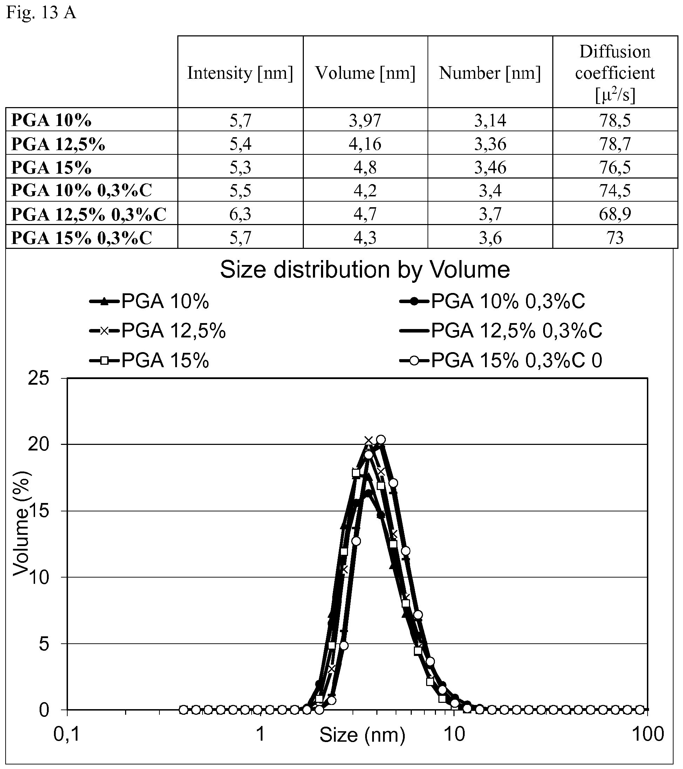

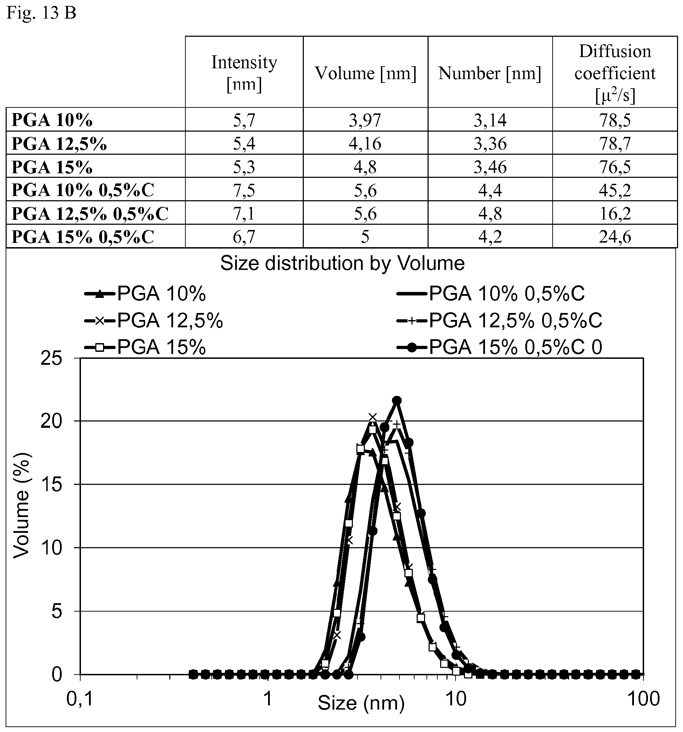

Figures 13A and B: the first figures show the mean micellar diameter values obtained by dynamic light scattering analysis expressed in the form of intensity, volume and number of samples respectively of 10% PGA, 12.5% PGA and 15% PGA with concentrations of 0.3% w/w and 0.5% w/w PCA ethyl cocoyl arginate. The second figures show the volumetric distribution curves of the same formulations.

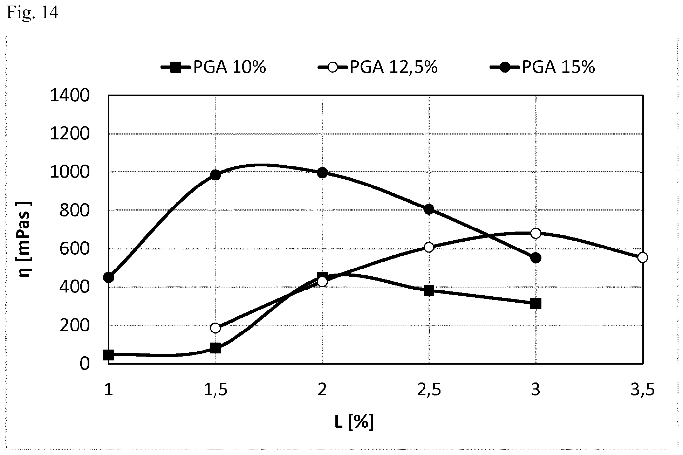

Figure 14: shows the graph of the viscosity trend at a shear rate of 46.4 s⁻¹ of 10% PGA, 12.5% PGA and 15% PGA and with increasing concentrations of sodium lactate (L).

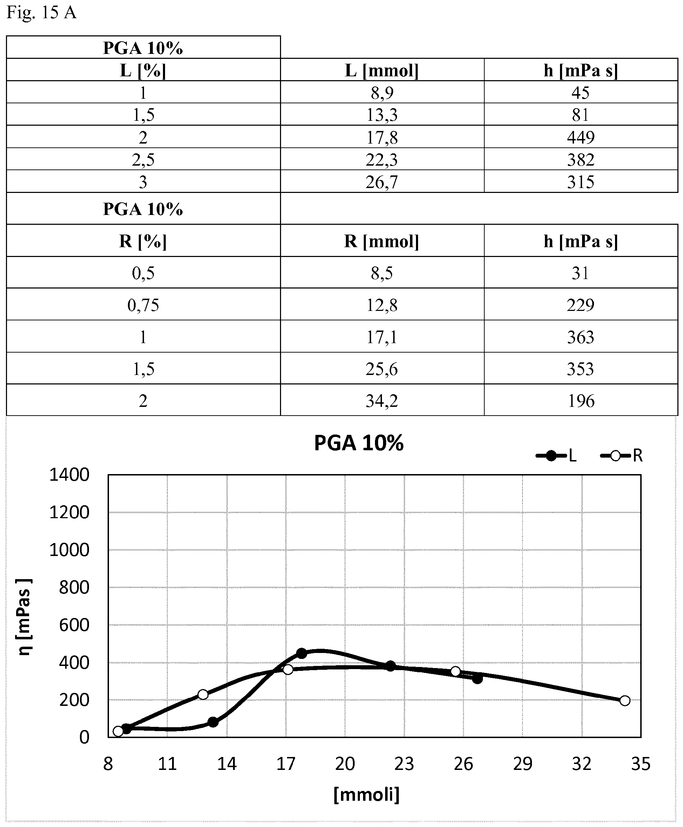

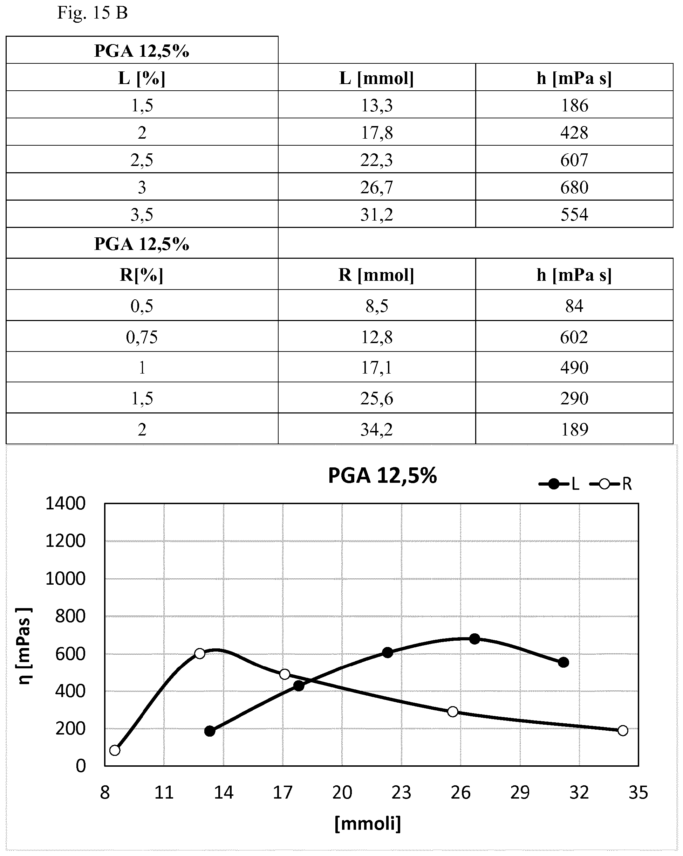

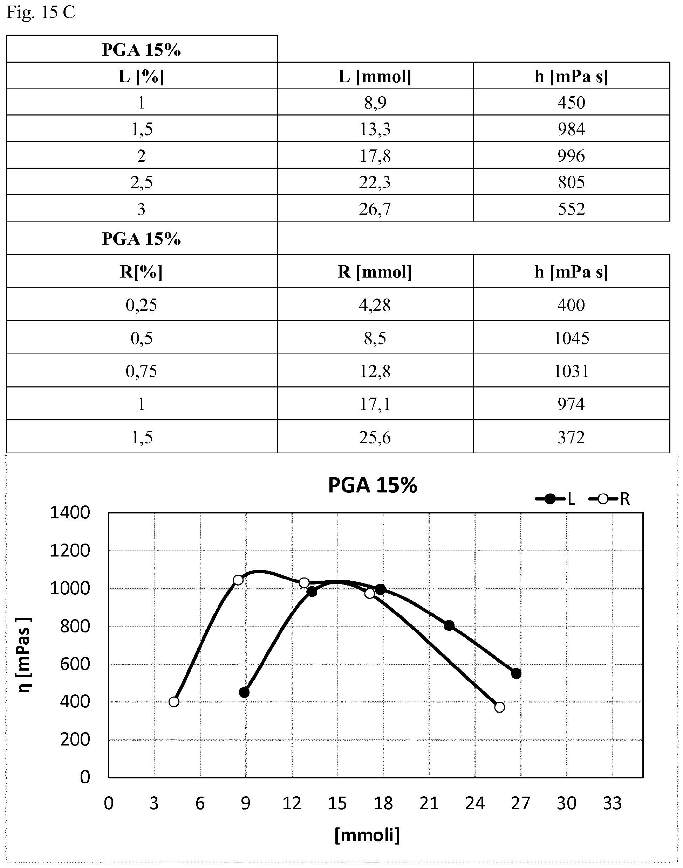

Figures 15A-C: show the graph of the viscosity trend at a shear rate of 46.4 s⁻¹ of 10% PGA, 12.5% PGA and 15% PGA with increasing concentrations of sodium chloride and sodium lactate expressed in millimoles.

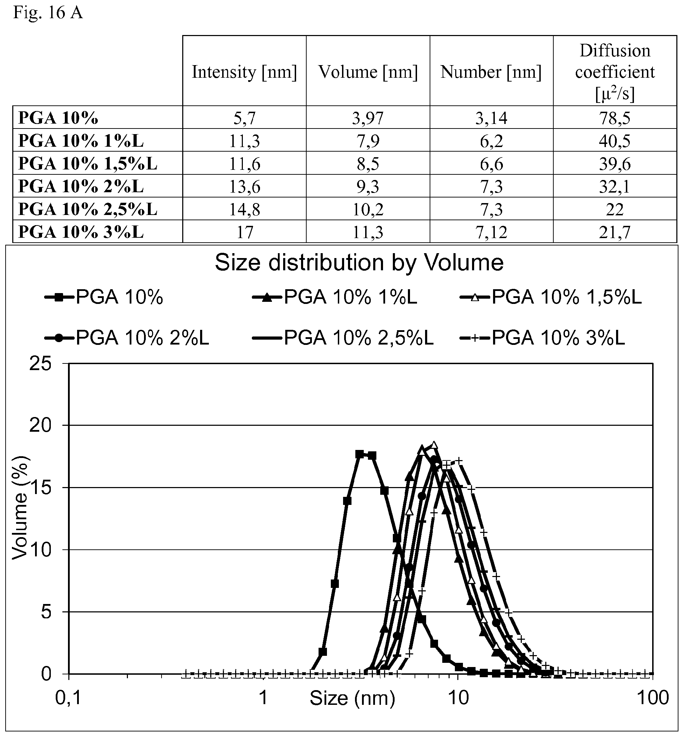

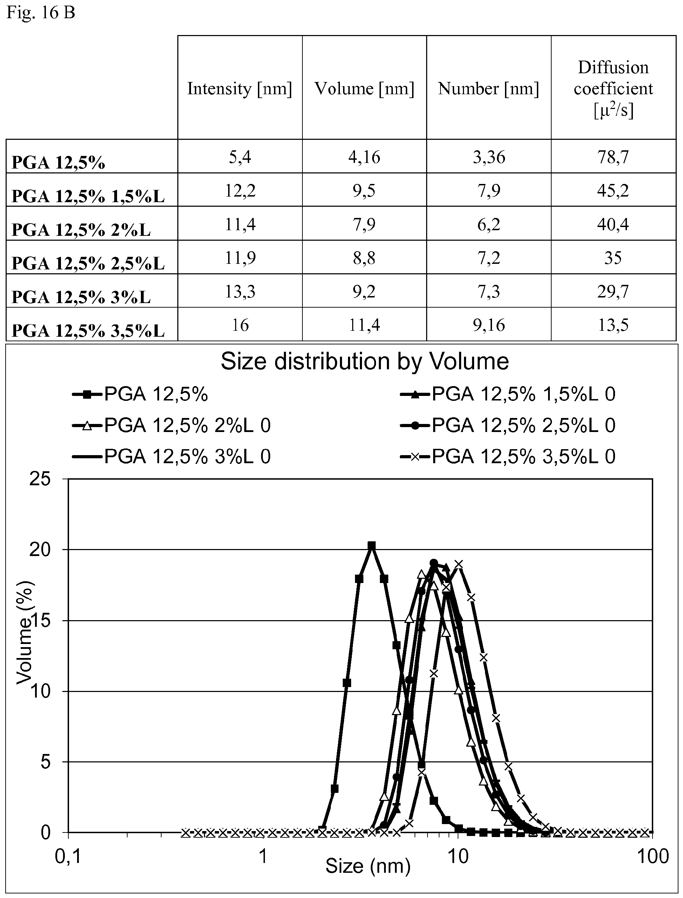

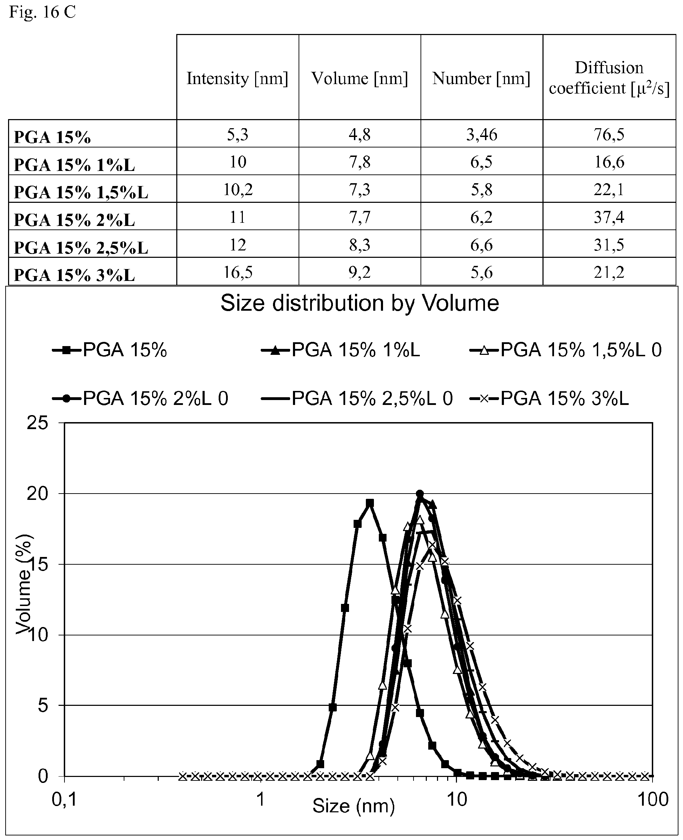

Figures 16A-C: the first figures show the mean micellar diameter values obtained by dynamic light scattering analysis expressed in the form of intensity, volume and number of samples respectively of 10% PGA, 12.5% PGA and 15% PGA with increasing concentrations of sodium lactate. The second figures show the volumetric distribution curves of the same formulations.

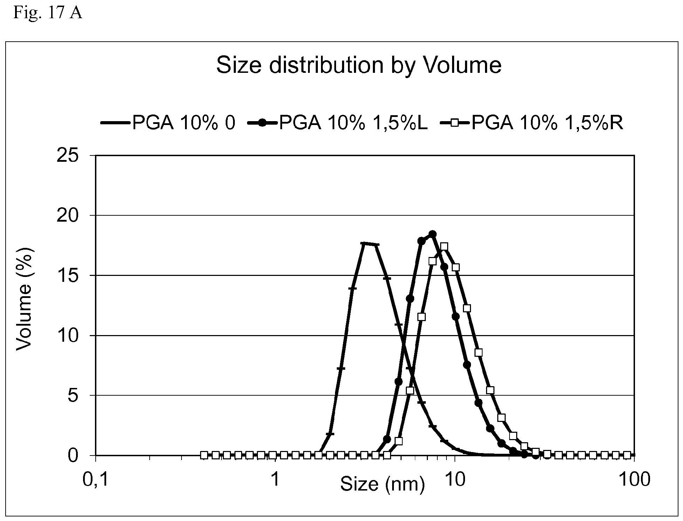

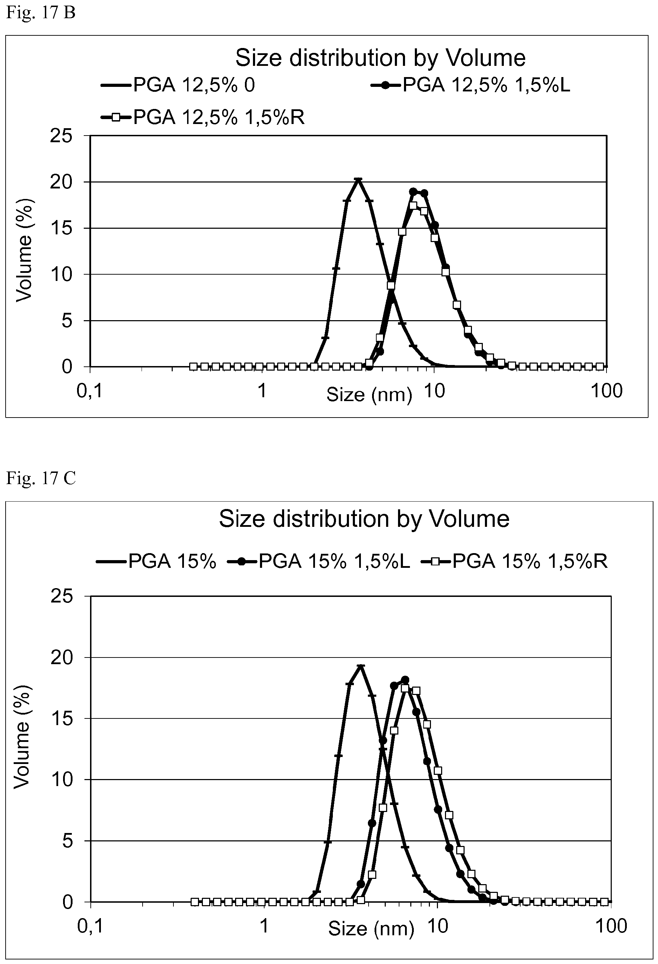

Figure 17: shows the volumetric distribution curves respectively of 10% PGA (Figure 17A), 12.5% PGA (Figure 17B) and 15% PGA (Figure 17C) alone and in the presence respectively of 1.5% sodium lactate and 1.5% sodium chloride.

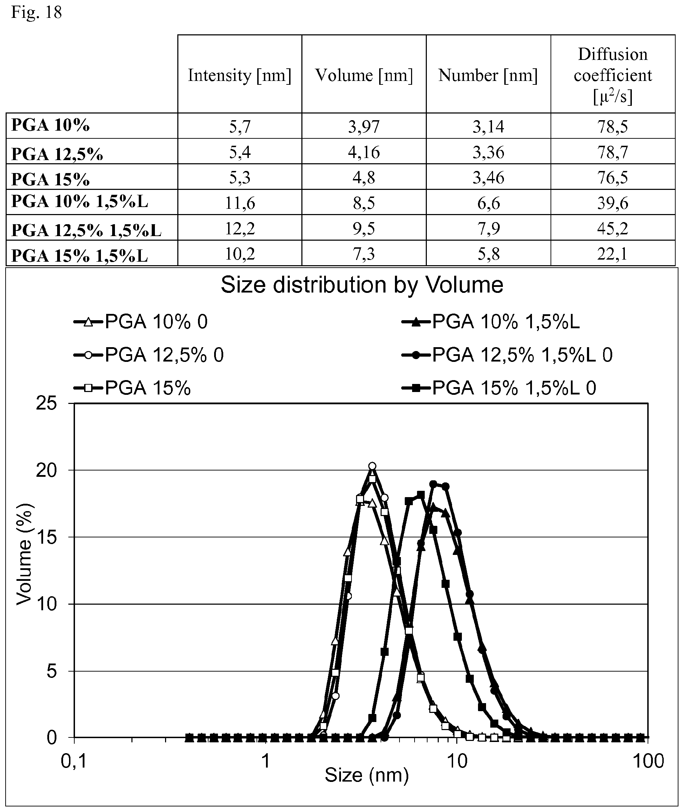

Figure 18: the first figure shows the mean micellar diameter values obtained by dynamic light scattering analysis expressed in the form of intensity, volume and number of samples respectively of 10% PGA, 12.5% PGA and 15% PGA with 1.5% sodium lactate. The second figure shows the volumetric distribution curve of the same formulations.

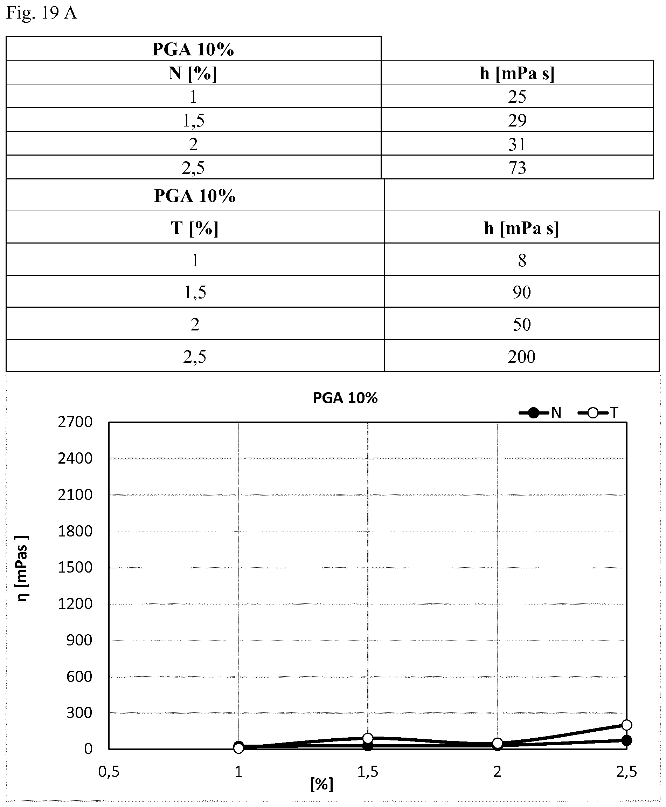

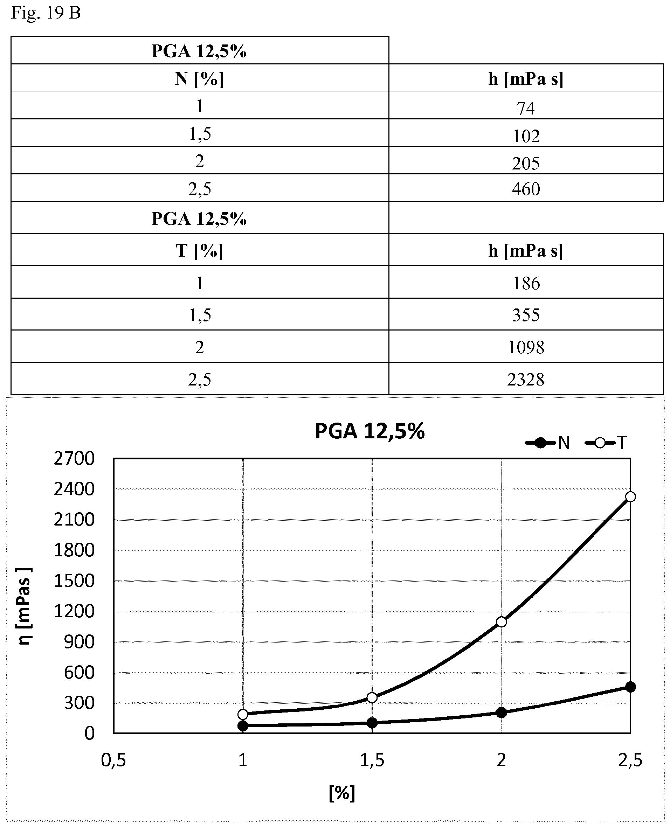

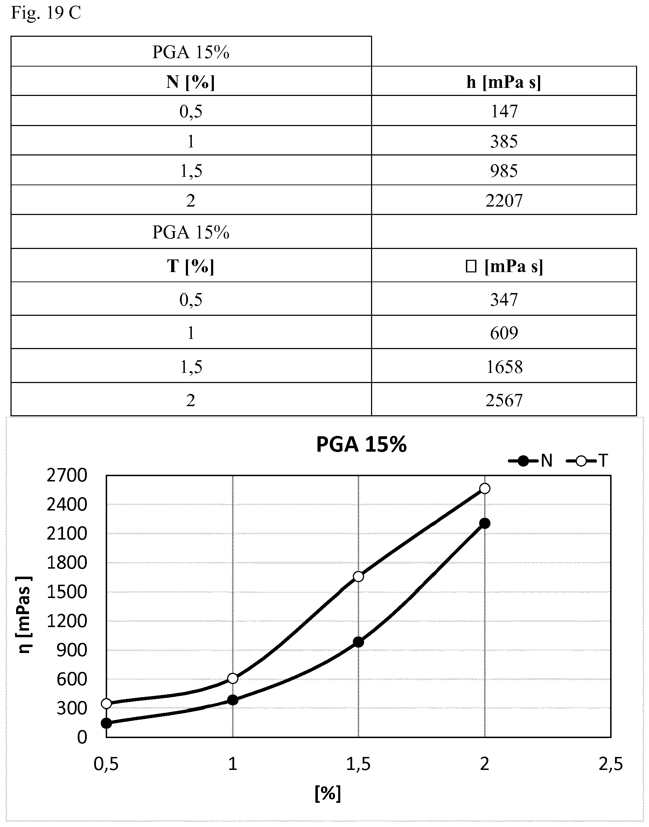

Figures 19A-C: the first figures are tables that report the viscosity values at a shear rate of 46.4 s⁻¹ of 10% PGA, 12.5% PGA and 15% PGA with increasing concentrations of PEG 55 propylene glycol oleate (N) and PEG 120 methyl glucose trioleate (T). The second figures show the viscosity trend at the same shear rate of the same formulations.

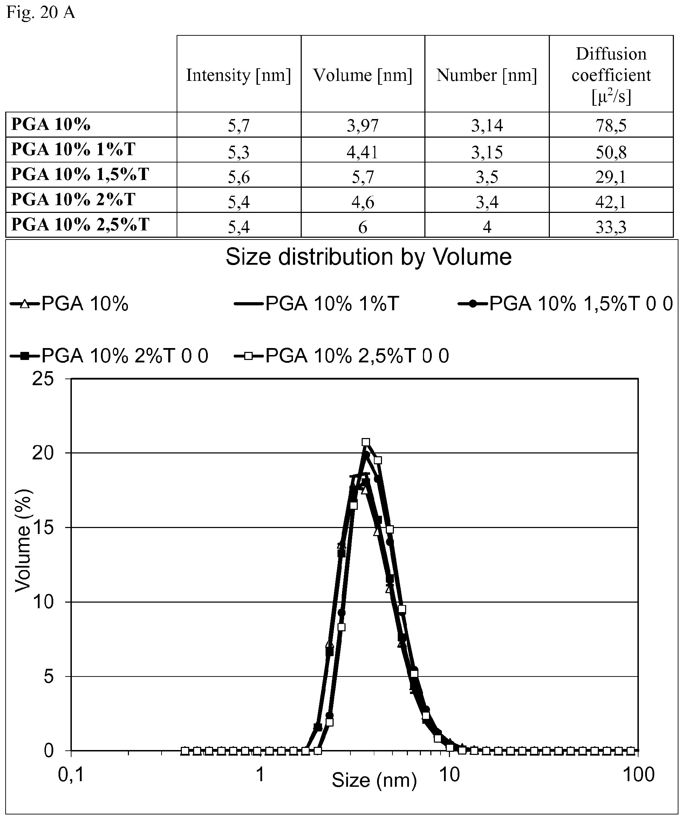

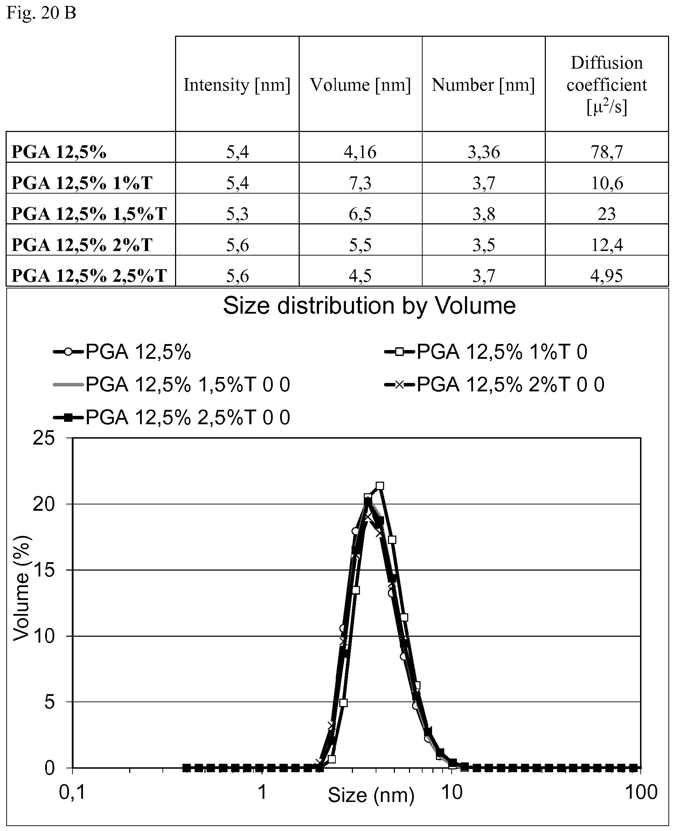

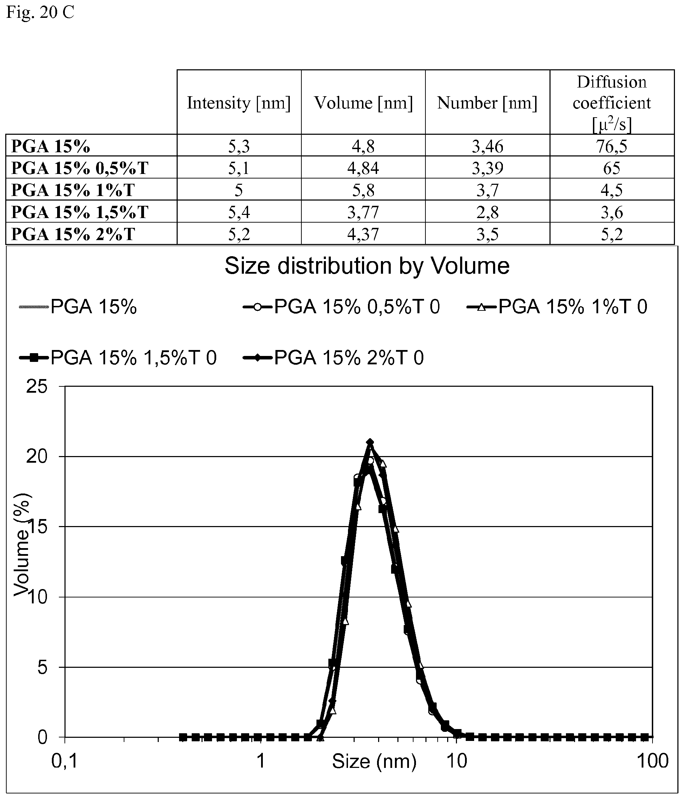

Figures 20A-C: the first figures show the mean micellar diameter values obtained by dynamic light scattering analysis expressed in the form of intensity, volume and number of samples respectively of 10% PGA, 12.5% PGA and 15% PGA with increasing concentrations of PEG 120 methyl glucose trioleate (T). The second figures show the volumetric distribution curves of the micellar diameter of the same formulations.

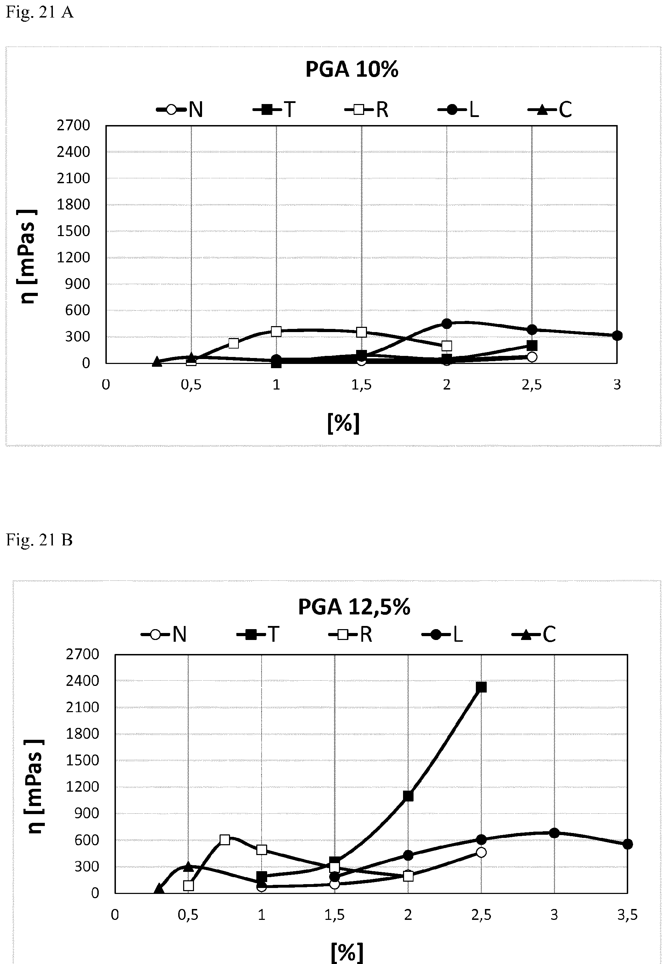

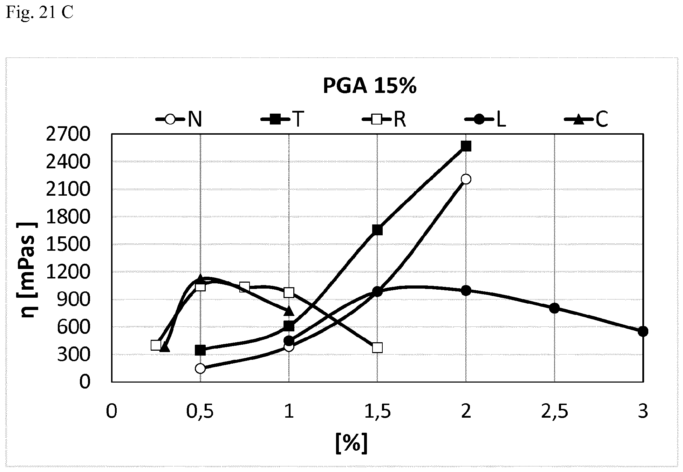

Figures 21A-C: show the viscosity trend at a shear rate of 46.4 s⁻¹ of 10% PGA, 12.5% PGA and 15% PGA with increasing concentrations of N, T, R, L and C.

### DETAILED DESCRIPTION OF THE INVENTION

For the purposes of the present invention, the verbs "comprise" and "contain" do not exclude the presence of further components in addition to those listed after them.

Preferably, the concentration of the component d, namely of PCA ethyl cocoyl arginate, is between 0.2 and 0.4%, even more preferably it is 0.3% by weight on the total weight of the composition.

The total concentration by weight of the active washing substances or components a -c) on the total weight of the detergent composition is preferably comprised between 9 and 15%, more preferably between 10 and 12.5%.

Preferably the ratio (a/b/c)/c) is 1.2.

The details of the present invention will become even more evident from the following experimental part, which is reported in the present description for illustrative but not limitative purposes.

### 1-EXPERIMENTAL PART

### 1.1. MATERIALS AND METHODS

This chapter lists the raw materials used for the creation of detergent systems and the methods of formulation and characterization of the obtained samples.

### 1.1.1 Materials

The used raw materials are:

### Surfactants:

INCI name: Sodium cocoyl glycinate, Water
Active washing substance: 30%

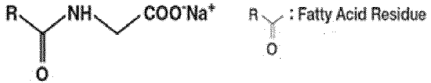

*Sodium cocoyl glycinate* is a non-ethoxylated primary anionic surfactant derived from coconut oil and the amino acid glycine. It has an excellent foaming power and is stable only in neutral-alkaline conditions. It is used at a concentration of up to 20% of the total in detergent formulations.
INCI name: Sodium cocoyl alaninate, Water
Active washing substance: 30%
INCI name: Cocoyl methyl glucamide, Water
Active washing substance: 40%

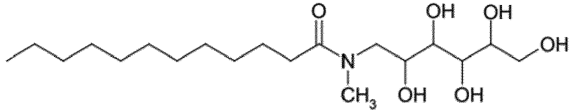

This non-ionic surfactant has foaming and conditioning properties. It has an excellent ecological profile and is particularly suitable for sensitive skin.

It has excellent viscosizing capabilities at low salt concentrations and is structuring for the surfactant system. It is compatible with all types of surfactants and is used at a concentration of 1-20%.
INCI name: Laureth-7 citrate
Active washing substance: 100%

   CH₃(CH₂)₁₀CH₂- [-OCH₂CH₂-]₇ -OC₆H₇O₆

*Laureth-7 citrate* is an anionic surfactant, chemically derived from the reaction of a lauryl alcohol molecule with ethylene oxide molecules: the number reported in laureth-7 corresponds to the number of inserted ethylene oxide molecules. The result is an amphiphilic molecule in which the lipophilic component is made of lauryl alcohol, a linear alcohol with 12 carbon atoms, whereas the hydrophilic component is made of ethylene oxide molecules. It is also esterified with the citric acid molecule. It is used at a concentration of 1-5% and imparts an acidic pH to the surfactant mixture in which it is inserted. This surfactant has known antibacterial properties against *Propionibacterium Acnes*, which is involved in acne disorder. Moreover, these properties are not modified if the surfactant is in a mixture with other components (Mehling et al., 2008).
- INCI name: PCA ethyl cocoyl arginate

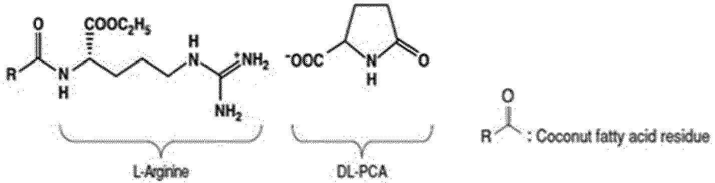

### Active washing substance: 100%

*PCA ethyl cocoyl arginate* is a biodegradable cationic surfactant derived from L-arginine. It is stable at acidic pHs, but is neutralized in an alkaline and basic environment. It also shows an antimicrobial activity against *Propionibacterium Acnes* at a pH lower than 7. In vitro cytotoxicity evaluations determine its gentleness on the skin.
- INCI name: Lauroyl arginine
   Active washing substance: 100%

### Humectants:

PROPYLENE GLYCOL
INCI name: Propylene glycol

### Rheological modifiers:

SODIUM CHLORIDE
INCI name: Sodium chloride
SODIUM LACTATE
INCI name: Sodium lactate 60%, Water
INCI name: PEG-120 Methyl Glucose Trioleate 40%, Propylene Glycol, Water
INCI name: PEG-55 Propylene Glycol Oleate 40%, Propylene Glycol

### PH regulators:

CITRIC ACID
INCI name: Citric acid

### 1.1.2 Method chosen to identify the composition that meets the requirements and the obtained results

The first part of the study was focused on the selection of a mixture of surfactants with intrinsically gentle characteristics and on their optimal ratio to maintain a 15% active washing substance. Then it was evaluated the insertion of functional agents with conditioning action derived from arginine within the system with 15% SAL, which allow improving the after-feel of the wash and bring the skin back to optimal charge conditions.

The active washing substance was reduced to 12.5% and 10% to make the surfactant system *milder.* Then viscosizing agents belonging to different chemical categories were added to optimize the mixture in terms of stability and to ensure the application of the product on the skin.

All formulations have been subjected to:
- centrifugal test to evaluate their mechanical stability,
- rheological analyses, under *Constant Controlled Shear Rate,* to determine the viscous properties after the insertion of different concentrations of viscosifying and conditioning agents,
- dynamic light scattering analyses to monitor the size of the micelles in solution. The most interesting samples in terms of application were subjected to accelerated ageing in a stove thermostated at 40°C to assess its stability over time.

- Preparation of detergent systems with 15% active washing substance

The detergent systems were prepared by using mixtures of a non-ionic derivative (Cocoyl methyl glucamide) at a concentration of 5% and two different 6% anionic surfactants: Sodium cocoyl glycinate and Sodium cocoyl alaninate. The mixture of surfactants containing Sodium cocoyl glycinate one day after the preparation precipitated to a pH > 5, whereas the detergent system in which Sodium cocoyl alaninate was present gave rise to a stable, homogeneous and transparent mixture, with a rich and creamy foam.

In a second step, the addition of the anionic surfactant Laureth-7 citrate used as active principle against acne at a concentration of 4% was evaluated, as indicated in the technical data sheet. This surfactant belongs to the category of alkyl ether citrates, compounds used as antimicrobials that have the advantage of not altering microflora and skin pH (Mehlign et al., 2008).

The preparation of the prototypes was carried out using a 200 ml beaker, a technical, an analytical weighing scale and a heated plate equipped with a magnetic stirrer.

All samples were prepared in a standard amount of 100 grams, sufficient to perform all rheological, stability and dynamic light scattering tests.

The investigated concentration ranges and the used preparation methods have been determined thanks to a critical reading of the technical data sheets of each material used.

The detergents are prepared as follows: the surfactants *Laureth-7 citrate* **(P)**, cocoyl methyl glucamide (**G**) and sodium cocoyl alaninate (**A**) are added in sequence to the aforementioned 200 ml beaker containing preserved water, and then mixed with a magnetic stirrer until obtaining a clear solution. The surfactant mixture was prepared in different ratios of active washing substance, thus obtaining four systematic compositions (Formulation 1A, 1B with a constant 15% SAL as shown in the following table).

### Formulations 1A, 18, 1C and 1D

| | **P0 G7 A8** | **P3 G6 A6** | **P4 G5 A6** | **P4 G6 A5** |
|---|---|---|---|---|
| | % | % | % | % |
| *Laureth-7 citrate **(P)*** | 0 | 3 | 4 | 4 |
| *Cocoyl methyl glucamide **(G)*** | 7 | 6 | 5 | 6 |
| *Sodium cocoyl alaninate **(A)*** | 8 | 6 | 6 | 5 |

All formulations were analysed by dynamic light scattering using ZETASIZER NANO-ZS by MALVERN.

Figure 1 shows the mean micellar diameter values obtained through the aforementioned analysis in the form of intensity, volume and number of the aforementioned formulations and the graph shows the distribution curves of the micellar diameter in volume of samples in different ratios.

The above formulations were subsequently added with 0.5% w/w sodium chloride, an electrolyte commonly used to increase the viscosity of surfactant-based formulations. Their rheological properties were evaluated by constant controlled shear rate measurements, conducted in a shear rate range between 4 and 150 s⁻¹ with a RheoPlus Anton Paar MCR 101 rheometer.

Figure 2 shows the viscosity curve as a function of the shear rate of the detergent systems, obtained by showing in the graph the viscosity values at equilibrium, calculated as the average value of each measurement stage.

The viscosity profile of the Laureth-7 citrate anionic surfactant-free system is of the Newtonian type, the viscosity remains constant as the applied shear rate increases and shows the lowest values (around 100 mPas). On the other hand, in samples in which the anionic surfactant is present, where it is therefore possible to have an interaction between the charges in solution and the charges on the micelle surface, with a consequent reduction of the mobility of these latter, a weakly pseudo-plastic behaviour is observed at high shear rates and with absolute viscosity values higher than a decade.

The sample with the highest viscosity, around 1000 mPas, is the one with a P4 G5 A6 ratio, whereas if higher or lower concentrations of anionic surfactant are used, corresponding to an increase or a decrease of the charge on the micelle surface, the obtained viscosity values are similar, but lower than those of the sample P4 G5 A6. Dimensional characterization through dynamic light scattering analysis of the same prototypes is shown in Figure 3.

As previously observed, in the absence of the anionic surfactant Laureth-7 citrate (P0 G7 A8, Formulation 1A) the formed micelles have a larger particle size than the other prototypes. The addition of sodium chloride in all detergent systems increases micelle size if compared to detergent systems that do not contain it.

The P4 G5 A6 system has been selected as the optimal ratio with 15% SAL, subsequently named 15% PGA, for the best viscosity yield after the addition of sodium chloride. The behaviour of this mixture was subsequently analysed by reducing the active washing substance and inserting conditioning and viscosizing agents into the considered system.
- Insertion of the conditioning agent Lauroyl arginine (Y) and PCA ethyl cocoyl arginate (C) in the detergent system with 15% SAL

A greasy skin detergent needs a conditioning action supported by molecules that are able to improve the after-feel of the wash, bringing the skin back to its optimal charge conditions and having no surfatting effect. The conditioning agents are normally cationic or amphoteric molecules, since the presence of a positively charged group allows the molecule to neutralize the negative charges of keratin exposed during the wash. These molecules have the ability of settling on the skin surface thus having a film-forming action. Among the different conditioning agents available on the cosmetic market, two derivatives of arginine were selected: Lauroyl arginine and PCA ethyl cocoyl arginate.

Besides being a conditioning agent, Lauroyl arginine also acts as a foam stabilizer in detergent formulations, but is poorly soluble and to be inserted in a formulation it needs to be pre-dissolved at 80°C in propylene glycol before being added to the surfactant system.

Therefore, also in this case the insertion of *Lauroyl arginine* **(Y)** in the 15% PGA samples involves a pre-solubilization in propylene glycol and heating up to 80°C under stirring to be then added to the detergent system in the final location.

The final formulation in this case is the Formulation 2 shown in the following table Formulation 2

| | 15% PGA 0.3%Y |
|---|---|
| | % |
| Preserved water | q.s. to 100 |
| *Laureth -7 citrate **(P)*** | 4 |
| *Cocoyl methyl glucamide **(G)*** | 5 |
| *Sodium cocoyl alaninate **(A)*** | 6 |
| *Propylenglycol **(I)*** | 3.5 |
| ***Lauroyl arginine (Y)*** | 0,3 |

*PCA ethyl cocoyl arginate* has an antimicrobial activity against *Proprionibacterium Acnes* in an acidic pH range, which makes it even more interesting in the cosmetic field for the formulation of an acneic skin cleanser. Both molecules were added at a concentration of 0.3% w/w to the detergent system though maintaining a constant 15% active substance.

The final Formulation 3 has the same formulation as Formulation 2 with the only difference that instead of lauroyl arginine it contains the same content of PCA ethyl cocoyl arginate, indicated with the abbreviation C.

The addition of lauroyl arginine at a concentration of 0.3% w/w does not lead to changes in the system pH and, as shown by dynamic light scattering analysis, does not change the size of the micelles of the detergent.

Its inclusion in the detergent system added with a rheological modifier (0.5% w/w sodium chloride (R)) determines a net decrease of the viscosity of the sample (Figure 4) from values of 1000 mPas to 500 mPas due to the presence of the propylene glycol necessary for its solubilisation.

The insertion of the cationic surfactant of amino acid derivation PCA ethyl cocoyl arginate does not require particular technical measures. It is added at concentrations of 0.3%, 0.5% and 1% w/w for simple solubilisation at 45°C together with the other surfactants. At the highest concentration (1% w/w) of PCA ethyl cocoyl arginate, however, a clouding of the solution is observed, which makes the dynamic light scattering analysis impossible.

The addition of PCA ethyl cocoyl arginate (C) to the detergent system progressively increases the diameter of the micelles as its concentration increases, even if in a very low variation range (Figure 5), but drastically decreases the diffusion coefficient.

The effects of this molecule on the system are also evident from the data obtained by means of the rheological analysis in Constant Controlled Shear Rate, conducted one week after the preparation of the samples, wherein the prototype contains 0.5% w/w PCA ethyl cocoyl arginate (C) shows a viscosity (Figure 6) higher than 1000 mPas, comparable with the previously verified one, obtained by adding 0.5% of sodium chloride.

The contribution of the two conditioned agents examined to the detergent system with 15% SAL is very different: only PCA ethyl cocoyl arginate positively modifies the viscous properties of the mixture, thus turning out as a molecule with a significant technical role.
- Cleansing systems with 15%, 12.5% and 10% active washing substance

The active washing substance was reduced to 12.5% (12.5% PGA) and 10% (10% PGA), though maintaining the same percentage ratios among the components of the mixture as shown in the following table:

| | **PGA 15%** | **PGA 12,5%** | **P GA 10%** |
|---|---|---|---|
| | % | % | % |
| *Laureth- 7 citrate **(P)*** | 4 | 3,33 | 2,67 |
| *Cocoyl methyl glucamide **(G)*** | 5 | 4,17 | 3,33 |
| *Sodium cocoyl alaninate **(A)*** | 6 | 5 | 4 |

The samples having a 5.5 pH were analysed by means of Dynamic Light Scattering analysis and the results, shown in Figure 7, indicate the absence of significant variations from the dimensional point of view with respect to the 15% SAL sample.

This behaviour is in agreement with the fact that even in the presence of lower SALs the detergent is still concentrated, because the ratio among the different surfactants composing remains unchanged.

Viscosity is a key property for a detergent product, because it determines the application properties, both for dispensing it from the container and distributing it on the skin and/or hair. Furthermore, in the detergent products the insertion of viscosizing agents and therefore the study of their rheological properties allows the improvement of the physical-chemical stability among the components of the surfactant mixture.

### Insertion of viscosizing agents into 15, 12.5 and 10% SAL systems

The effects of rheological modifiers have therefore been studied according to the SAL of detergent systems by using different chemical molecules: inorganic and organic electrolytes (sodium chloride, PCA ethyl cocoyl arginate, sodium lactate) and non-ionic thickeners (polyethylene glycol derivatives).

### Sodium chloride

The first viscosizing agent inserted into gentle surfactant systems is sodium chloride: it is an inorganic compound commonly used in detergent formulations for its high yield and low cost. Its concentration within the detergent products varies according to the mixture of used surfactants and must be experimentally determined (usually it must be lower than 3% w/w). The concentration range investigated for the sample preparation was 0.25% to 2% w/w and the viscosity curves were obtained by means of a Constant Controlled Shear Rate analysis. The following tables and Figure 8 show a comparison among the viscosity values at a shear rate of 46.4 s⁻¹ based on the sodium chloride concentrations obtained for each SAL. Figure 9 compares the viscosity values at a shear rate of 46.4 s⁻¹ based on the sodium chloride concentrations obtained for each SAL.
(a)

| **PGA** 1**0%** | | |
|---|---|---|
| **R [%]** | **R [mmol]** | **η [mPa s]** |
| 0,5 | 8,5 | 31 |
| 0,75 | 12,8 | 229 |
| 1 | 17,1 | 363 |
| 1,5 | 25,6 | 353 |
| 2 | 34,2 | 196 |

(b)

| **PGA 12,5%** | | |
|---|---|---|
| **R[%]** | **R [mmol]** | **η [mPa s**] |
| 0,5 | 8,5 | 84 |
| 0,75 | 12,8 | 602 |
| 1 | 17,1 | 490 |
| 1,5 | 25,6 | 290 |
| 2 | 34,2 | 189 |

(c)

| **PGA 15%** | | |
|---|---|---|
| **R[%]** | **R [mmol]** | **η [mPa s]** |
| 0,25 | 4,28 | 400 |
| 0,5 | 8,5 | 1045 |
| 0,75 | 12,8 | 1031 |
| 1 | 17,1 | 974 |
| 1,5 | 25,6 | 372 |

The analysis shows that the highest viscosity values, around 1000 mPas, are obtained with a 15% SAL (15% PGA). In particular, the curve trend shows a plateau between 0.5% and 0.75 % w/w sodium chloride. For concentrations of lower surfactants (12.5% - 10% SAL), the viscosity curves are decidedly lower and a higher concentration of electrolyte is required, respectively 0.75% w/w to 1% w/w, to reach the highest viscosity level.

The samples were characterized by Dynamic Light Scattering analysis (Figure 10A-10C) to monitor the size of the micelles of the surfactants in solution after the insertion of the electrolyte.

The data reported in Figures 10A-10C show that the higher the concentration of sodium chloride, the larger the micelle size in the samples in all three systems. In fact, sodium chloride competes with the surfactants in solution, thus reducing the charge density of the micelles. The addition of sodium chloride shields the repulsive forces between the polar groups of surfactants due to its electrostatic properties, produces an increase in the volume of the micelles and a decrease in the diffusion coefficient as the electrolyte is placed outside them (Demissie H., Duraismay R., Effect of electrolytes on the surface and micellar characteristics of Sodium dodecyl sulphate surfactant solutions, Journal of Scientific & Innovative Research (2016), 5 (6): 208-214.).

A comparison of the results of the analysis of samples with a concentration of sodium chloride (R) of 0.5% w/w but different SAL shows that the prototype with 10% active washing substance has a larger micelle size (Figure 11).

### PCA-ethyl cocoyl arginate

In the 15% SAL system (Figure 6), the conditioning agent PCA ethyl cocoyl arginate has also shown a significant effect as a rheological modifier. This molecule was then inserted at the same concentrations (0.3%, 0.5% and 1 % w/w) also in prototypes with less active washing substance (12.5% - 10%). The viscosity curves shown in Figure 12 were obtained by rheological analysis in constant controlled shear rate, showing the graph of the viscosity values at a shear rate of 46.4 s⁻¹ as reported in the following table by varying the PCA ethyl cocoyl arginate (C) and the active washing substance concentrations.
(a)

| **PGA 10%** | |
|---|---|
| **C[%]** | **η [mPa s]** |
| 0,3 | 22 |
| 0,5 | 67 |
| 1 | 30 |

(b)

| **PGA 12,5%** | |
|---|---|
| **C [%]** | **η [mPa s]** |
| 0,3 | 60 |
| 0,5 | 301 |
| 1 | 120 |

(c)

| **PGA 15%** | |
|---|---|
| **C [%]** | **η [mPa s]** |
| 0,3 | 385 |
| 0,5 | 1120 |
| 1 | 777 |

The viscosity trend shows that the rheological effects induced by this molecule depend on the initial viscosity of the sample, which in turn is strictly dependent on the active washing substance. The two prototypes with active washing substance of 10% (10% PGA) and 12.5% (12.5% PGA) achieve lower viscosity values than the system with the highest surfactant concentration (15% PGA) at all concentrations investigated, although for all systems there is a viscosity peak at a 0.5% concentration of PCA ethyl cocoyl arginate.

Samples with a concentration of 1% w/w PCA ethyl cocoyl arginate rapidly show problems of instability due to the poor solubility of the cationic surfactant inside the mixture, so they have been excluded from the systematic as regards the Dynamic Light Scattering analysis.

From the graph relating to size distribution by volume (Figures 13A-13B), differences in micelle size can be detected for samples with concentration of conditioning agent of 0.5% w/w in all three SALs investigated (as previously verified for 15% SAL), whereas if added to a concentration of 0.3% w/w, the PCA ethyl cocoyl arginate does not produce significant effects.

### Sodium lactate

Sodium lactate is the sodium salt of lactic acid and thanks to its remarkable ability to retain water and to its compatibility with the skin, it is used in cosmetics as a moisturizing agent, a pH regulator and a preservative.

This multifunctional organic compound, normally used at a concentration between 1% and 5% w/w, is a natural hydration factor already present in the skin and allows the stratum corneum to perform its barrier function against the external environment. In detergent products it can also have the function of viscosizing agent, but being an organic salt, it is less hydrophilic, has a lower solvation capacity and its electrostatic charge has a reduced efficiency if compared to inorganic salts such as sodium chloride.

Sodium lactate was introduced in a concentration range between 1% and 3.5% w/w in detergent systems with different active washing substance and its viscosizing properties were determined by constant controlled shear rate measurements. Taking as reference the viscosity values at a shear rate of 46.4 s⁻¹ as reported in the following table, it was possible to identify the concentration with the best viscosity yield as reported in Figure 14.

The viscosity graph based on the sodium lactate (R) concentration in the samples with different SAL shows that, unlike what had been detected for sodium chloride, the best yield in terms of viscosity is obtained with 2% w/w sodium lactate.
(a)

| **PGA 10%** | | |
|---|---|---|
| **L [%]** | **L [mmol]** | **η [mPa s]** |
| 1 | 8.9 | 45 |
| 1,5 | 13,3 | 81 |
| 2 | 17,8 | 449 |
| 2,5 | 22.3 | 382 |
| 3 | 26,7 | 315 |

(b)

| **PGA 12,5%** | | |
|---|---|---|
| **L [%]** | **L [mmol]** | **η [mPa s]** |
| 1,5 | 13,3 | 186 |
| 2 | 17,8 | 428 |
| 2,5 | 22,3 | 607 |
| 3 | 26.7 | 680 |
| 3,5 | 31.2 | 554 |

(c)

| **PGA 15%** | | |
|---|---|---|
| **L [%]** | **L [mmol]** | **η [mPa s]** |
| 1 | 8.9 | 450 |
| 1,5 | 13,3 | 984 |
| 2 | 17,8 | 996 |
| 2,5 | 22,3 | 805 |
| 3 | 26.7 | 552 |

The differences in the effects between the two salts, since they have different molecular weights and charges, are better highlighted by the viscosity value graphs depending on the used salt millimoles (Figure 15A-15C).

Using the Dynamic Light Scattering System it has also been compared the particle size of the samples with 15%, 12.5%, and 10% SAL by varying the sodium lactate concentration present in the mixtures (Figures 16A-16C). The results indicate an increase in the average distribution of the micelles size in Volume as a function of the salt concentration at all the investigated SALs, analogously to what already highlighted with the inorganic type electrolyte (sodium chloride).

By comparing the curves respectively obtained with sodium chloride and sodium lactate at the same weight concentration (1.5%) in detergent mixtures with different SALs (Figure 17), completely comparable trends have been observed: both salts remarkably increase the volume of the micelles if compared to samples that do not contain them.

As already highlighted by the viscosity data, also the results by Dynamic Light Scattering show that the addition of sodium lactate in the diluted systems (12.5% - 10% SAL) is more efficient than it is in the system with 15% active washing substance. Sodium lactate significantly increases micelle size if compared to the corresponding reference systems, and the effect is particularly greater in the diluted samples, as shown in Figure 18.

Samples containing sodium lactate and sodium chloride at all investigated SALs were unstable at thermal stress in an oven at 40°C after one month.

### Polyethylene glycol derivatives

Polyethylene glycol derivatives are non-ionic surfactants mainly used as viscosizing agents. They consist of a small polar head and of long lipophilic tails that are arranged inside the micellar structure, thus forming a stable network thanks to the intermolecular associations that favour a sudden viscosity increase. The present work takes into consideration PEG-55 propylene glycol oleate and PEG-120 methyl glucose trioleate: the first has only one long lipophilic chain, whereas the second is made up of four chains. PEG-55 propylene glycol oleate (N) was inserted in a concentration range comprised between 0.5% and 2.5% w/w, whereas PEG-120 methyl glucose trioleate (T) had a range comprised between 1% and 3% w/w, as indicated by the respective technical data sheets, in all systems with 10%, 12.5% and 15% active washing substance.

Starting from the viscosity values at a shear rate of 46.4 s⁻¹, viscosity trends based on the rheological modifier concentration considered respectively at 10%, 12.5% and 15% SAL (Figures 19A-19C) were obtained. In the most diluted detergent (10% SAL) neither of the two polyethylene glycol derivatives have an effect on viscosity, whereas significant values have been found in systems with 12.5% and 15% active washing substance by increasing the added concentrations.

In particular, the addition of 2% w/w PEG-120 methyl glucose trioleate to the system with 15% SAL and of 2.5% w/w of the same rheological modifier to the system with 12.5% SAL allows reaching a viscosity higher than 2000 mPas.

Due to its surfactant nature and to its significant steric hindrance, PEG-120 methyl glucose trioleate fits into the micellar system without modifying its size, as shown by the dynamic light scattering analysis (Figure 20A-C) in samples with 10%, 12.5% and 15% SAL.

Moreover, PEG-120 methyl glucose trioleate does not compromise the transparency of detergent systems and even their foaming properties, thus proving to be very efficient as a viscosizing agent and stabilizer in the formulations of gentle detergents at high concentrations. The samples containing PEG-120 methyl glucose trioleate were stable at the thermal stress in an oven at 40°C after one month.

The viscosity curves obtained with the different rheological modifiers investigated for each detergent system (SAL 10% - 12.5% - 15%) are shown below (Figures 21A-21C).

If the viscosity-increasing mechanism of the systems is of the electrolytic type, the rheological effects can also be measured in diluted systems and the micelle size of the surfactants in the detergent system increases considerably as the electrolyte concentration increases (Figure 18). When the rheological modifier instead works at the micelle interface, the surfactant concentration must exceed a threshold concentration (SAL > 10) so that the effects are visible. Moreover, due to their surfactant nature, these derivatives do not modify the micelle size (Figures 20A-20C).

It therefore results that the formulation of detergent systems based on intrinsically gentle surfactants and low active washing substance (SAL 10%) is critical in terms of viscosity and long-term stability.

### 1.1.3 Conclusions

This study allowed identifying a very gentle and skin-friendly surfactant mixture, formed by a glucoside derivative (*Cocoyl Methyl Glucamide*), one of an amino acid type (*Sodium Cocoyl Alaninate*) and one of an anionic type (*Laureth-7 citrate*). The study also allowed determining the optimal concentration ratios and the percentages of active washing substance at which surfactants should be used, also in relation to the rheological modifier concentrations of different chemical nature included in the system.

The study further showed that the addition of sodium chloride and sodium lactate as well as of polyethylene glycol derivatives to the aforesaid surfactant mixture was not successful.

The study also highlighted how molecules proposed on the market as active ingredients, such as PCA ethyl cocoyl arginate, can instead have a significant technical role: its salt nature makes it able to confer rheological effects and variations in terms of micelle size similar to those detected, and quite surprising, of the electrolytes.

## Claims

1. Gentle detergent composition comprising:
a) sodium cocoyl alaninate;
b) cocoyl methyl glucamide;
c) laureth-7 citrate and
d) PCA ethyl cocoyl arginate;
wherein:
i) a, b, c are the cleaning substances whose total concentration is between 8 and 18% by weight on the total weight of said composition;
ii) the ratio (a/b/c)/c is between 0.8 and 1.4;
iii) d is present in an amount ranging from 0.1 to 0.6% on the total weight of said composition.

2. Gentle detergent composition according to claim 1, wherein the concentration of the component d is between 0.2 and 0.4

3. Gentle detergent composition according to claim 2, wherein the concentration of the component d is 0.3% on the total weight of the composition.

4. Gentle detergent composition according to any one of the claims 1-3, wherein said ratio (a/b/c)/c is 1.2.

5. Gentle detergent composition according to any one of the claims 1-4, wherein the total concentration by weight of the active cleaning agents, i.e. the components a - c relative to the weight of the total detergent composition is between 9 and 15%.

6. Gentle detergent composition according to any one of claims 1-5, wherein the total concentration by weight of the active washing agents, i.e. the components a - c relative to the weight of the total detergent composition is between 10 and 12.5%.

7. Use of the compositions according to any one of the claims 1-6 for cleansing greasy skin.

8. The compositions according to any one of the claims 1-6 for use in cleansing acneic skin.

## Patentansprüche

1. Sanfte Waschmittelzusammensetzung, umfassend:
a) Natriumcocoylalaninat,
b) Cocoylmethylglucamid;
c) Laureth-7-Citrat und
d) PCA-Ethylcocoylarginat,
wobei:
i) a, b, c die Reinigungsmittel sind, deren Gesamtkonzentration zwischen 8 und 18 Gew.-%, bezogen auf das Gesamtgewicht der Zusammensetzung, liegt;
ii) das Verhältnis (a/b/c)/c zwischen 0,8 und 1,4 liegt;
iii) d in einer Menge in einem Bereich von 0,1 bis 0,6 % des Gesamtgewichts der Zusammensetzung vorliegt.

2. Sanfte Waschmittelzusammensetzung nach Anspruch 1, wobei die Konzentration der Komponente d zwischen 0,2 und 0,4 liegt.

3. Sanfte Waschmittelzusammensetzung nach Anspruch 2, wobei die Konzentration der Komponente d 0,3 %, bezogen auf das Gesamtgewicht der Zusammensetzung, beträgt.

4. Sanfte Waschmittelzusammensetzung nach einem der Ansprüche 1 bis 3, wobei das Verhältnis (a/b/c)/c 1,2 ist.

5. Sanfte Waschmittelzusammensetzung nach einem der Ansprüche 1-4, wobei die Gesamtkonzentration der aktiven Reinigungsmittel, d.h. der Komponenten a - c, bezogen auf das Gewicht der gesamten Waschmittelzusammensetzung, zwischen 9 und 15 Gew.-% liegt.

6. Sanfte Waschmittelzusammensetzung nach einem der Ansprüche 1-5, wobei die Gesamtkonzentration der aktiven Waschmittel, d. h. der Komponenten a - c, bezogen auf das Gewicht der gesamten Waschmittelzusammensetzung, zwischen 10 und 12,5 Gew.-% liegt.

7. Verwendung der Zusammensetzungen nach einem der Ansprüche 1-6 zum Reinigen von fettiger Haut.

8. Zusammensetzungen nach einem der Ansprüche 1-6 zur Verwendung bei der Reinigung von Haut mit Akne.

## Revendications

1. Composition détergente douce comprenant:
a) sodium cocoyl glycinate ;
b) cocoyl méthyl glucamide;
c) laureth-7 citrate et
d) arginate d'éthyle cocoyle PCA ;
dans laquelle :
i) a, b, c sont les substances nettoyantes dont la concentration totale est comprise entre 8 et 18 % en poids par rapport au poids total de ladite composition ;
ii) le rapport (a/b/c)/c est compris entre 0,8 et 1,4 ;
iii) d est présent en une quantité allant de 0,1 à 0,6 % par rapport au poids total de ladite composition.

2. Composition détergente douce selon la revendication 1, dans laquelle la concentration du composant d est comprise entre 0,2 et 0,4

3. Composition détergente douce selon la revendication 2, dans laquelle la concentration du composant d est de 0,3 % par rapport au poids total de la composition.

4. Composition détergente douce selon l'une quelconque des revendications 1 à 3, dans laquelle ledit rapport (a/b/c)/c est de 1,2.

5. Composition détergente douce selon l'une quelconque des revendications 1 à 4, dans laquelle la concentration totale en poids des agents nettoyants actifs, c'est-à-dire les composants a à c par rapport au poids de la composition détergente totale, est comprise entre 9 et 15 %.

6. Composition détergente douce selon l'une quelconque des revendications 1 à 5, dans laquelle la concentration totale en poids des agents de lavage actifs, c'est-à-dire les composants a à c par rapport au poids de la composition détergente totale, est comprise entre 10 et 12,5%.

7. Utilisation des compositions selon l'une quelconque des revendications 1 à 6 pour le nettoyage de la peau grasse.

8. Compositions selon l'une quelconque des revendications 1 à 6, destinées à être utilisées pour nettoyer la peau acnéique.
